(19) Europäisches Patentamt
European Patent Office
Office européen des brevets

(11) **EP 3 673 787 A1**

(12) **EUROPEAN PATENT APPLICATION**
published in accordance with Art. 153(4) EPC

(43) Date of publication:
01.07.2020 Bulletin 2020/27

(51) Int Cl.:
A61B 1/06 (2006.01)
A61B 1/045 (2006.01)
G02B 23/24 (2006.01)
G02B 23/26 (2006.01)

(21) Application number: 18849023.9

(22) Date of filing: 14.08.2018

(86) International application number:
PCT/JP2018/030291

(87) International publication number:
WO 2019/039354 (28.02.2019 Gazette 2019/09)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 23.08.2017 JP 2017159977

(71) Applicant: FUJIFILM Corporation
Tokyo 106-8620 (JP)

(72) Inventor: KURAMOTO, Masayuki
Ashigarakami-gun
Kanagawa 258-8538 (JP)

(74) Representative: Klunker IP
Patentanwälte PartG mbB
Destouchesstraße 68
80796 München (DE)

(54) **LIGHT SOURCE DEVICE AND ENDOSCOPE SYSTEM**

(57) There are provided a light source device and an endoscope system that can switch plural kinds of illumination light without a user's instruction.

A light source unit (20) emits first illumination light that includes a first red-light wavelength range and is used to emphasize a first blood vessel and second illumination light that includes a second red-light wavelength range and is used to emphasize a second blood vessel different from the first blood vessel. A light source control unit (21) performs control to cause each of the first illumination light and the second illumination light to be emitted for a light emission period of at least two or more frames and to automatically switch the first illumination light and the second illumination light.

FIG. 11

**Description**

**BACKGROUND OF THE INVENTION**

1. Field of the Invention

The present invention relates to a light source device and an endoscope system that switch and emit plural kinds of illumination light.

2. Description of the Related Art

In recent years, an endoscope system comprising a light source device, an endoscope, and a processor device has been widely used in a medical field. In the endoscope system, an object to be observed is irradiated with illumination light from an endoscope, and the image of the object to be observed is displayed on a monitor on the basis of RGB image signals that are obtained in a case where the image of the object to be observed, which is being illuminated with the illumination light, is picked by an image pickup element of the endoscope.
Further, in recent years, the switching of the spectrum of illumination light to be used also has been performed according to an object to be observed. For example, in JP2016-179236A, a fluorescent body is irradiated with two kinds of light, that is, violet laser light and blue laser light to generate illumination light. In a case where superficial blood vessels distributed at relatively shallow positions in a mucous membrane are to be observed, the light emission ratio of the violet laser light is made to be higher than that of the blue laser light and an object to be observed is illuminated with illumination light where the ratio of the violet laser light is increased. In contrast, in a case where deep blood vessels distributed at deep positions in a mucous membrane are to be observed, the light emission ratio of the blue laser light is made to be higher than that of the violet laser light and an object to be observed is illuminated with illumination light where the ratios of green fluorescence and red fluorescence are increased.

**SUMMARY OF THE INVENTION**

In recent years, a diagnosis focusing on a plurality of blood vessels having different depths, such as superficial blood vessels and deep blood vessels, has been made in an endoscopic field. The images of the plurality of blood vessels having different depths need to be displayed in such a diagnosis. For example, in the case of JP2016-179236A, a user operates an illumination light-changeover switch to switch illumination light for emphasizing superficial blood vessels and illumination light for emphasizing deep blood vessels and to display an image where the superficial blood vessels are emphasized and an image where the deep blood vessels are emphasized. However, in a case where the switching of the illumination light is performed by a user as described above, there is a problem that a shift in an observation position is likely to occur at the time of the switching of the illumination light. Accordingly, there is a request for a technique for switching plural kinds of illumination light without a user's instruction to display the images of the plurality of blood vessels having different depths. In addition, there is also a request for a technique for reducing a sense of incongruity that is to be given to a user due to the switching of plural kinds of illumination light.
An object of the invention is to provide a light source device and an endoscope system that can switch plural kinds of illumination light without a user's instruction.
A light source device according to an aspect of the invention comprises a light source unit and a light source control unit. The light source unit emits first illumination light including a first red-light wavelength range and used to emphasize a first blood vessel and second illumination light including a second red-light wavelength range and used to emphasize a second blood vessel different from the first blood vessel. The light source control unit causes each of the first illumination light and the second illumination light to be emitted for a light emission period of at least two or more frames and automatically switches the first illumination light and the second illumination light.
It is preferable that the first illumination light has a peak in a range of 400 nm to 440 nm. It is preferable that an intensity ratio of the second illumination light is higher than that of the first illumination light in at least one of wavelengths of 540 nm, 600 nm, or 630 nm.
It is preferable that the light source unit emits third illumination light different from the first illumination light and the second illumination light and the light source control unit causes the third illumination light to be emitted at a timing of the switching of the first illumination light and the second illumination light. It is preferable that the light source control unit includes a light emission period-setting unit setting a light emission period of the first illumination light and a light emission period of the second illumination light. It is preferable that the first illumination light includes a first green-light wavelength range and a first blue-light wavelength range in addition to the first red-light wavelength range and the second illumination light includes a second green-light wavelength range and a second blue-light wavelength range in addition to the second red-light wavelength range.

An endoscope system according to another aspect of the invention comprises the light source device according to the aspect of the invention, an image acquisition unit, and a display unit. The image acquisition unit acquires a first image obtained from image pickup of an object to be observed illuminated with the first illumination light and a second image obtained from image pickup of the object to be observed illuminated with the second illumination light. The display unit displays the first image and the second image as color images or monochrome images.

An endoscope system according to another aspect of the invention comprises the light source device according to the aspect of the invention, an image acquisition unit, and a specific color adjustment section. The image acquisition unit acquires a first image obtained from image pickup of an object to be observed illuminated with the first illumination light and a second image obtained from image pickup of the object to be observed illuminated with the second illumination light. The specific color adjustment section adjusts colors of the first image and the second image. Further, the specific color adjustment section causes a color of a mucous membrane included in the first image or a color of a mucous membrane included in the second image to match a target color. It is preferable that the endoscope system further comprises a portion setting section setting a portion being observed and the specific color adjustment section causes the color of the mucous membrane included in the first image or the color of the mucous membrane included in the second image to match a target color corresponding to the portion.

An endoscope system according to another aspect of the invention comprises the light source device according to the aspect of the invention, an image acquisition unit, a specific color adjustment section, and a tone instruction-receiving unit. The image acquisition unit acquires a first image obtained from image pickup of an object to be observed illuminated with the first illumination light and a second image obtained from image pickup of the object to be observed illuminated with the second illumination light. The specific color adjustment section adjusts colors of the first image and the second image. The color adjustment instruction-receiving unit receives a color adjustment instruction related to adjustment of a color of a mucous membrane, a color of the first blood vessel, or a color of the second blood vessel from a user. The specific color adjustment section adjusts the color of the mucous membrane, the color of the first blood vessel, or the color of the second blood vessel according to the color adjustment instruction.

An endoscope system according to another aspect of the invention comprises the light source device according to the aspect of the invention, an image acquisition unit, and a specific color adjustment section. The image acquisition unit acquires a first image obtained from image pickup of an object to be observed illuminated with the first illumination light and a second image obtained from image pickup of the object to be observed illuminated with the second illumination light. The specific color adjustment section adjusts colors of the first image and the second image. Further, the specific color adjustment section causes a color of a mucous membrane included in the first image to coincide with a color of a mucous membrane included in the second image.

An endoscope system according to another aspect of the invention comprises the light source device according to the aspect of the invention, an image acquisition unit, and a color extension processing section. The image acquisition unit acquires a first image obtained from image pickup of an object to be observed illuminated with the first illumination light and a second image obtained from image pickup of the object to be observed illuminated with the second illumination light. The color extension processing section performs color extension processing for increasing a difference between a plurality of ranges included in the object to be observed on the first image and the second image. It is preferable that the endoscope system further comprises a portion setting section setting a portion being observed and a result of the color extension processing is adjusted using an adjustment parameter determined for each portion.

An endoscope system according to another aspect of the invention comprises the light source device according to the aspect of the invention, an image acquisition unit, a frequency emphasis section, and an image combination section. The image acquisition unit acquires a first image obtained from image pickup of an object to be observed illuminated with the first illumination light and a second image obtained from image pickup of the object to be observed illuminated with the second illumination light. The frequency emphasis section obtains a frequency-emphasized image, in which a frequency component corresponding to a specific range included in the object to be observed is emphasized, from the first image and the second image. The image combination section combines the first image or the second image with the frequency-emphasized image to obtain the first image or the second image which has been subjected to structure emphasis processing in which the specific range is subjected to structure emphasis. It is preferable that the endoscope system further comprises a portion setting section setting a portion being observed and a pixel value of the first image or the second image having been subjected to the structure emphasis processing is adjusted using an adjustment parameter determined for each portion.

According to the invention, it is possible to switch plural kinds of illumination light without a user's instruction.

**BRIEF DESCRIPTION OF THE DRAWINGS**

Fig. 1 is a diagram showing the appearance of an endoscope system according to a first embodiment.

Fig. 2 is a block diagram showing the functions of the endoscope system according to the first embodiment.

Fig. 3 is a graph showing the emission spectra of violet light V, blue light B, green light G, and red light R.

Fig. 4 is a graph showing the emission spectrum of first illumination light, that includes violet light V, blue light B, green light G, and red light R.

Fig. 5 is a graph showing the emission spectrum of second illumination light that includes violet light V, blue light B, green light G, and red light R.

Fig. 6 is a diagram showing the light emission period of the first illumination light and the light emission period of the second illumination light.

Fig. 7 is a diagram showing a light emission period-setting menu.

Fig. 8 is a graph showing the emission spectrum of third illumination light that includes violet light V, blue light B, green light G, and red light R.

Fig. 9 is an image diagram showing a first special image.

Fig. 10 is an image diagram showing a second special image.

Fig. 11 is a diagram showing the switching display of a color first special image and a color second special image.

Fig. 12 is a diagram showing a third special image that is displayed at the time of the switching of the first special image and the second special image.

Fig. 13 is a diagram showing the switching display of a monochrome first special image and a monochrome second special image.

Fig. 14 is a block diagram showing a first special image processing unit and a second special image processing unit that uses a B/G ratio and a G/R ratio.

Fig. 15 is a diagram showing first to fifth ranges that are distributed in a signal ratio space.

Fig. 16 is a diagram showing a radius vector change range Rm.

Fig. 17 is a graph showing a relationship between a radius vector r and a radius vector Rx(r) subjected to saturation emphasis processing.

Fig. 18 is a diagram showing an angle change range Rn.

Fig. 19 is a graph showing a relationship between an angle $\theta$ and an angle Fx($\theta$) subjected to hue emphasis processing.

Fig. 20 is a diagram showing the distribution of the first to fifth ranges in the signal ratio space before and after saturation emphasis processing and hue emphasis processing.

Fig. 21 is a diagram showing the distribution of the first to fifth ranges in an ab space before and after saturation emphasis processing and hue emphasis processing.

Fig. 22 is a block diagram showing the functions of a structure emphasis section that uses a B/G ratio and a G/R ratio.

Fig. 23 is a table showing a relationship among combination ratios g1(B/G ratio, G/R ratio) to g4g1(B/G ratio, G/R ratio) of a B/G ratio and a G/R ratio.

Fig. 24 is a flowchart showing the flow of a multi-observation mode.

Fig. 25 is a block diagram showing the functions of a first special image processing unit and a second special image processing unit that use color difference signals Cr and Cb.

Fig. 26 is a diagram showing the first to fifth ranges that are distributed in a CrCb space.

Fig. 27 is a diagram showing the distribution of the first to fifth ranges in the CrCb space before and after saturation emphasis processing and hue emphasis processing.

Fig. 28 is a block diagram showing the functions of the structure emphasis section that uses color difference signals Cr and Cb.

Fig. 29 is a block diagram showing the functions of a first special image processing unit and a second special image processing unit that use a hue H and a saturation S.

Fig. 30 is a diagram showing the first to fifth ranges that are distributed in an HS space.

Fig. 31 is a diagram showing the distribution of the first to fifth ranges in the HS space before and after saturation emphasis processing and hue emphasis processing.

Fig. 32 is a block diagram showing the functions of the structure emphasis section that uses a hue H and a saturation S.

Fig. 33 is a block diagram showing the functions of an endoscope system according to a second embodiment.

Fig. 34 is a graph showing the emission spectrum of normal light.

Fig. 35 is a graph showing the emission spectrum of first illumination light.

Fig. 36 is a graph showing the emission spectrum of second illumination light.

## DESCRIPTION OF THE PREFERRED EMBODIMENTS

[First embodiment]

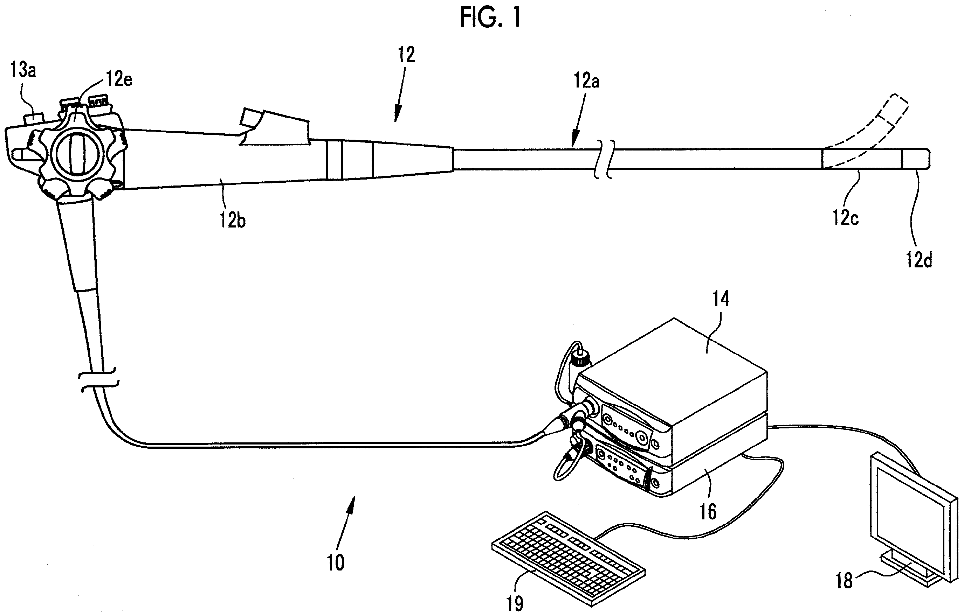

As shown in Fig. 1, an endoscope system 10 according to a first embodiment includes an endoscope 12, a light source device 14, a processor device 16, a monitor 18, and a console 19. The endoscope 12 is optically connected to the light source device 14, and is electrically connected to the processor device 16. The endoscope 12 includes an insertion part 12a that is to be inserted into an object to be examined, an operation part 12b that is provided at the proximal end portion of the insertion part 12a, and a bendable part 12c and a distal end part 12d that are provided on the distal end side of the insertion part 12a. In a case where angle knobs 12e of the operation part 12b are operated, the bendable part 12c is operated to be bent. As the bendable part 12c is operated to be bent, the distal end part 12d faces in a desired direction. The console 19 includes a mouse and the like in addition to a keyboard shown in Fig. 1.

Further, the operation part 12b is provided with a mode changeover SW 13a in addition to the angle knobs 12e. The mode changeover SW 13a is used for an operation for switching a normal observation mode, a first special observation mode, a second special observation mode, and a multi-observation mode. The normal observation mode is a mode where a normal image is displayed on the monitor 18. The first special observation mode is a mode where a first special image in which superficial blood vessels (first blood vessel) are emphasized is displayed on the monitor 18. The second special observation mode is a mode where a second special image in which deep blood vessels (second blood vessel) are emphasized is displayed on the monitor 18. The multi-observation mode is a mode where the first special image and the second special image are automatically switched and displayed on the monitor 18. A foot switch may be used as a mode switching unit, which is used to switch a mode, other than the mode changeover SW 13a.

The processor device 16 is electrically connected to the monitor 18 and the console 19. The monitor 18 outputs and displays image information and the like. The console 19 functions as a user interface (UI) that receives an input operation, such as function settings. An external recording unit (not shown), which records image information and the like, may be connected to the processor device 16.

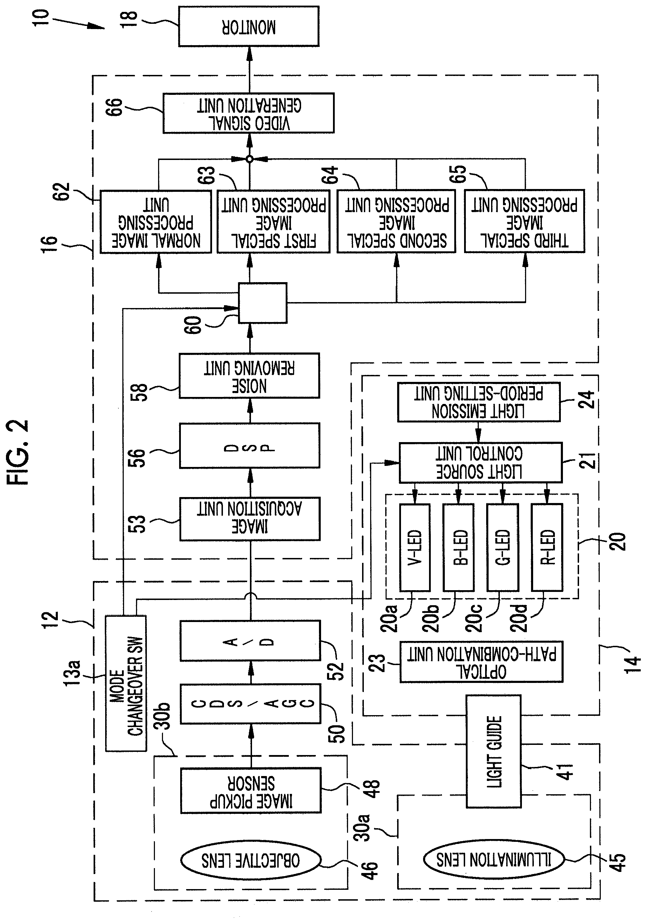

As shown in Fig. 2, the light source device 14 includes a light source unit 20, a light source control unit 21, and an optical path-combination unit 23. The light source unit 20 includes a violet light emitting diode (V-LED) 20a, a blue light emitting diode (B-LED) 20b, a green light emitting diode (G-LED) 20c, and a red light emitting diode (R-LED) 20d. The light source control unit 21 controls the drive of the LEDs 20a to 20d. The optical path-combination unit 23 combines the optical paths of pieces of light that are emitted from the four color LEDs 20a to 20d and have four colors. The inside of an object to be examined is irradiated with the pieces of light, which are combined by the optical path-combination unit 23, through a light guide 41 inserted into the insertion part 12a and an illumination lens 45. A laser diode (LD) may be used instead of the LED.

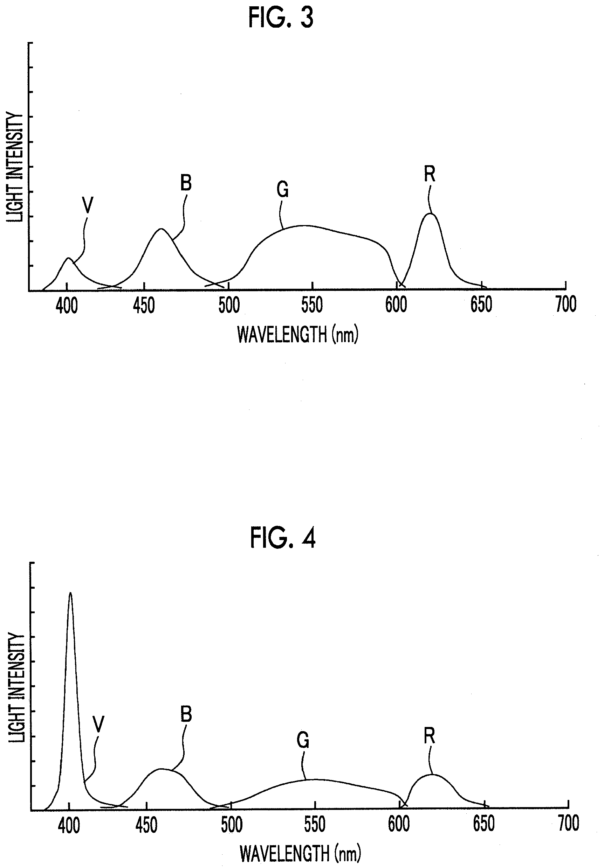

As shown in Fig. 3, the V-LED 20a generates violet light V of which the central wavelength is in the range of $405\pm10$ nm and the wavelength range is in the range of 380 to 420 nm. The B-LED 20b generates blue light B of which the central wavelength is in the range of $460\pm10$ nm and the wavelength range is in the range of 420 to 500 nm. The G-LED 20c generates green light G of which the wavelength range is in the range of 480 to 600 nm. The R-LED 20d generates red light R of which the central wavelength is in the range of 620 to 630 nm and the wavelength range is in the range of 600 to 650 nm.

The light source control unit 21 performs control to turn on the V-LED 20a, the B-LED 20b, the G-LED 20c, and the R-LED 20d in all observation modes. Further, the light source control unit 21 controls the respective LEDs 20a to 20d so that normal light of which the light intensity ratios of violet light V, blue light B, green light G, and red light R are Vc:Bc:Gc:Rc is emitted in the normal observation mode.

Furthermore, the light source control unit 21 controls the respective LEDs 20a to 20d so that first illumination light of which the light intensity ratios of violet light V, blue light B, green light G, and red light R are Vs1:Bs1:Gs1:Rs1 is emitted in the first special observation mode. To emphasize superficial blood vessels, it is preferable that the first illumination light has a peak in the range of 400 nm to 440 nm. For this purpose, the light intensity ratios Vs1:Bs1:Gs1:Rs1 of the first illumination light are set so that the light intensity of violet light V is higher than the light intensity of each of blue light B, green light G, and red light R as shown in Fig. 4 (Vs1>Bs1, Gs1, and Rs1). Further, since the first illumination light includes a first red-light wavelength range like red light R, the first illumination light can accurately reproduce the color of a mucous membrane. Furthermore, since the first illumination light includes a first blue-light wavelength range and a first green-light wavelength range like violet light V, blue light B, and green light G, the first illumination light can also emphasize various structures, such as glandular structures and unevenness, in addition to the above-mentioned superficial blood vessels.

Further, the light source control unit 21 controls the respective LEDs 20a to 20d so that second illumination light of which the light intensity ratios of violet light V, blue light B, green light G, and red light R are Vs2:Bs2:Gs2:Rs2 is emitted in the second special observation mode. To emphasize deep blood vessels, it is preferable that the intensity ratio of the second illumination light is higher than that of the first illumination light in at least one of wavelengths of 540 nm, 600 nm, or 630 nm.

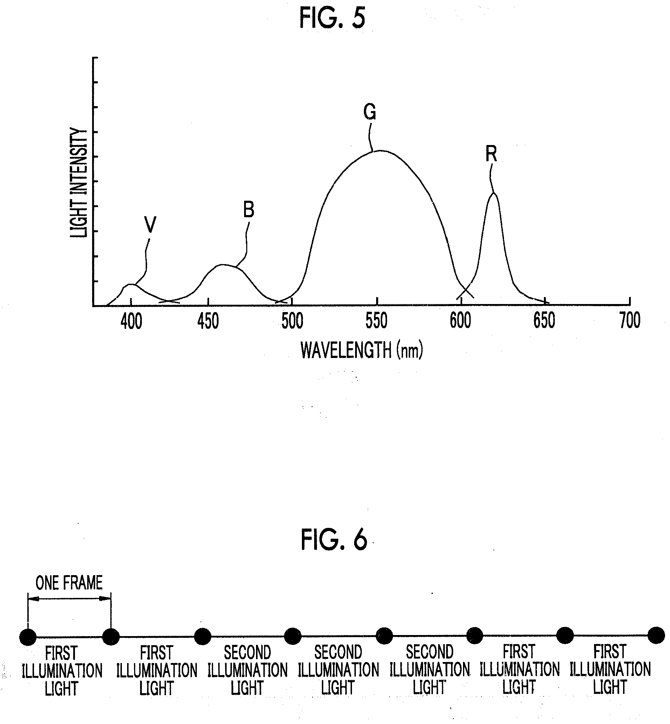

For this purpose, the light intensity ratios Vs2:Bs2:Gs2:Rs2 of the second illumination light are set so that the amount of green light G or red light R of the second illumination light is larger than the amounts of blue light B,: green light G, and red light R of the first illumination light as shown in Fig. 5. Further, since the second illumination light includes, a second red-light wavelength range like red light R, the second illumination light can accurately reproduce the color of a mucous membrane. Furthermore, since the second illumination light includes a second blue-light wavelength range and a second green-light wavelength range like violet light V, blue light B, and green light G, the second illumination light can also emphasize various structures, such as unevenness, in addition to the above-mentioned deep blood vessels.

In a case where a mode is set to the multi-observation mode, the light source control unit 21 performs control to emit each of the first illumination light and the second illumination light for a light emission period of two or more frames and to automatically switch and emit the first illumination light and the second illumination light. For example, in a case where the light emission period of the first illumination light is set to two frames and the light emission period of the second illumination light is set to three frames, the second illumination light continues to be emitted for three frames after the first illumination light continues to be emitted for two frames as shown in Fig. 6. Here, each of the light emission period of the first illumination light and the light emission period of the second illumination light is set to a period of at least two or more frames. The reason why each light emission period is set to a period of two or more frames as described above is that the illumination light of the light source device 14 is immediately switched but at least two or more frames are required to switch the image processing of the processor device 16. In addition, since there is a case where flicker occurs due to the switching of illumination light, each light emission period is set to a period of two or more frames to reduce a burden on an operator caused by flicker. "Frame" means a unit used to control an image pickup sensor 48 that picks up the image of an object to be observed. For example, "one frame" means a period including at least an exposure period where the image pickup sensor 48 is exposed to light emitted from an object to be observed and a read-out period where image signals are read out. In this embodiment, a light emission period is determined so as to correspond to "frame" that is a unit of image pickup.

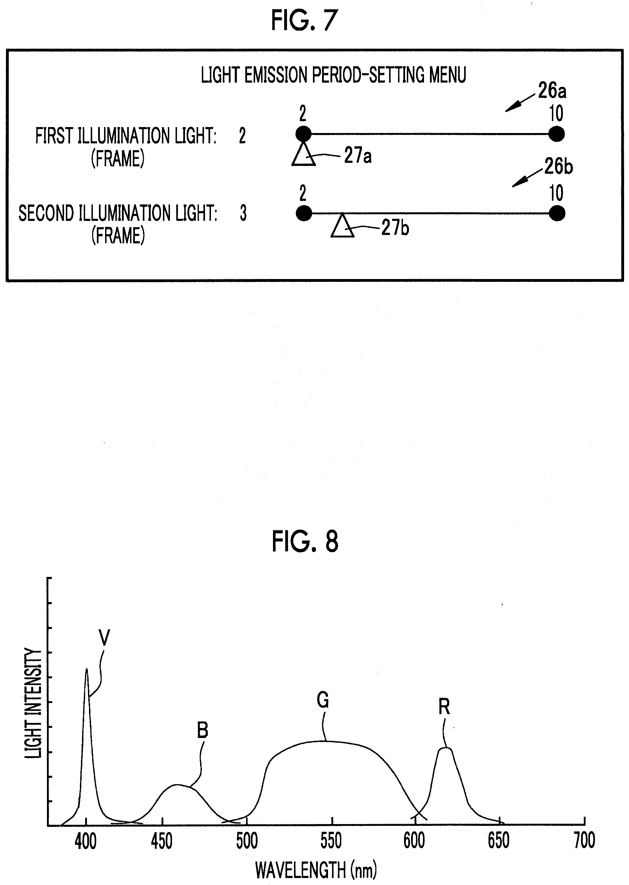

The light emission period of the first illumination light and the light emission period of the second illumination light can be appropriately changed by a light emission period-setting unit 24 that is connected to the light source control unit 21. In a case where an operation for changing a light emission period is received by the operation of the console 19, the light emission period-setting unit 24 displays a light emission period-setting menu shown in Fig. 7 on the monitor 18. The light emission period of the first illumination light can be changed between, for example, two frames and ten frames. Each light emission period is assigned to a slide bar 26a.

In a case where the light emission period of the first illumination light is to be changed, a user operates the console 19 to position a slider 27a at a position on the slide bar 26a that represents a light emission period to which the user wants to change a light emission period. Accordingly, the light emission period of the first illumination light is changed. Even in the case of the light emission period of the second illumination light, a user operates the console 19 to position a slider 27b at a position on a slide bar 26b (to which a light emission period in the range of two frames to ten frames is assigned) that represents a light emission period to which the user wants to change a light emission period. Accordingly, the light emission period of the second illumination light is changed.

In a case where a mode is set to the multi-observation mode, the light source control unit 21 may cause third illumination light, which is different from the first illumination light and the second illumination light, to be emitted at the time of the switching of illumination light to the second illumination light from the first illumination light or at a timing of the switching of illumination light to the first illumination light from the second illumination light. It is preferable that the third illumination light is emitted for at least one or more frames.

Further, it is preferable that light intensity ratios Vs3:Bs3:Gs3:Rs3 of the third illumination light are between the light intensity ratios Vs1:Bs1:Gs1:Rs1 of the first illumination light and the light intensity ratios Vs2:Bs2:Gs2:Rs2 of the second illumination light. For example, it is preferable that the light intensity ratios of the third illumination light are averages of the light intensity ratios of the first illumination light and the light intensity ratios of the second illumination light as shown in Fig. 8. That is, "Vs3=(Vs1+Vs2)/2", "Bs3=(Bs1+Bs2)/2", "Gs3=(Gs1+Gs2)/2", and "Rs3=(Rsl+Rs2)/2" are satisfied. In a case where the above-mentioned third illumination light is emitted at a timing of the switching of the first illumination light and the second illumination light, a sense of incongruity, such as a change in color, occurring at the time of the switching of illumination light is not given to a user.

As shown in Fig. 2, the light guide 41 is built in the endoscope 12 and a universal cord (a cord connecting the endoscope 12 to the light source device 14 and the processor device 16), and transmits the pieces of light, which are combined by the optical path-combination unit 23, to the distal end part 12d of the endoscope 12. A multimode fiber can be used as the light guide 41. For example, a thin fiber cable of which a total diameter of a core diameter of 105 $\mu$m, a cladding diameter of 125 $\mu$m, and a protective layer forming a covering is in the range of $\varphi$ 0.3 to 0.5 mm can be used.

The distal end part 12d of the endoscope 12 is provided with an illumination optical system 30a and an image pickup optical system 30b. The illumination optical system 30a includes an illumination lens 45, and an object to be observed is irradiated with light transmitted from the light guide 41 through the illumination lens 45. The image pickup optical system 30b includes an objective lens 46 and an image pickup sensor 48. Light reflected from the object to be observed is incident on the image pickup sensor 48 through the objective lens 46. Accordingly, the reflected image of the object to be observed is formed on the image pickup sensor 48.

The image pickup sensor 48 is a color image pickup sensor, and picks up the reflected image of an object to be examined and outputs image signals. It is preferable that the image pickup sensor 48 is a charge coupled device (CCD) image pickup sensor, a complementary metal-oxide semiconductor (CMOS) image pickup sensor, or the like. The image pickup sensor 48 used in the invention is a color image pickup sensor that is used to obtain RGB image signals corresponding to three colors of R (red), G (green), and B (blue), that is, a so-called RGB image pickup sensor that comprises R-pixels provided with R-filters, G-pixels provided with G-filters, and B-pixels provided with B-filters.

The image pickup sensor 48 may be a so-called complementary color image pickup sensor, which comprises complementary color filters corresponding to C (cyan), M (magenta), Y (yellow), and G (green), instead of an RGB color image pickup sensor. In a case where a complementary color image pickup sensor is used, image signals corresponding to four colors of C, M, Y, and G are output. Accordingly, the image signals corresponding to four colors of C, M, Y, and G need to be converted into image signals corresponding to three colors of R, G, and B by complementary color-primary color conversion. Further, the image pickup sensor 48 may be a monochrome image pickup sensor that includes no color filter. In this case, since the light source control unit 21 causes blue light B, green light G, and red light R to be emitted in a time-sharing manner, demosaicing needs to be added to the processing of image pickup signals.

The image signals output from the image pickup sensor 48 are transmitted to a CDS/AGC circuit 50. The CDS/AGC circuit 50 performs correlated double sampling (CDS) or auto gain control (AGC) on the image signals that are analog signals. The image signals, which have been transmitted through the CDS/AGC circuit 50, are converted into digital image signals by an analog/digital converter (A/D converter) 52. The digital image signals, which have been subjected to A/D conversion, are input to the processor device 16.

The processor device 16 corresponds to a medical image processing device that processes medical images, such as images obtained by the endoscope 12. The processor device 16 comprises an image acquisition unit 53, a digital signal processor (DSP) 56, a noise removing unit 58, a signal switching unit 60, a normal image processing unit 62, a first special image processing unit 63, a second special image processing unit 64, a third special image processing unit 65, and a video signal generation unit 66. Digital color image signals output from the endoscope 12 are input to the image acquisition unit 53. The color image signals are RGB image signals formed of R-image signals that are output from the R-pixels of the image pickup sensor 48, G-image signals that are output from the G-pixels of the image pickup sensor 48, and B-image signals that are output from the B-pixels of the image pickup sensor 48.

The DSP 56 performs various kinds of signal processing, such as defect correction processing, offset processing, gain correction processing, linear matrix processing, gamma conversion processing, and demosaicing processing, on the received image signals. Signals of defective pixels of the image pickup sensor 48 are corrected in the defect correction processing. Dark current components are removed from the RGB image signals having been subjected to the defect correction processing in the offset processing, so that an accurate zero level is set. The RGB image signals having been subjected to the offset processing are multiplied by a specific gain in the gain correction processing, so that signal levels are adjusted. The linear matrix processing for improving color reproducibility is performed on the RGB image signals having been subjected to the gain correction processing. After that, brightness or a saturation is adjusted by the gamma conversion processing. The demosaicing processing (also referred to as equalization processing or demosaicing) is performed on the RGB image signals having been subjected to the linear matrix processing, so that signals of colors deficient in each pixel are generated by interpolation. All the pixels are made to have the signals of the respective colors of R, G, and B by this demosaicing processing.

The noise removing unit 58 performs noise removal processing (for example, a moving-average method, median filtering, or the like) on the RGB image signals, which have been subjected to gamma correction and the like by the DSP 56, to remove noise from the RGB image signals. The RGB image signals from which noise has been removed are transmitted to the signal switching unit 60.

In a case where a mode is set to the normal observation mode by the mode changeover SW 13a, the signal switching unit 60 transmits the RGB image signals to the normal image processing unit 62. Further, in a case where a mode is set to the first special observation mode, the signal switching unit 60 transmits the RGB image signals to the first special image processing unit 63. Furthermore, in a case where a mode is set to the second special observation mode, the signal switching unit 60 transmits the RGB image signals to the second special image processing unit 64. Moreover in a case where a mode is set to the multi-observation mode, the RGB image signals obtained from illumination using the first illumination light and image pickup are transmitted to the first special image processing unit 63, the RGB image signals obtained from illumination using the second illumination light and image pickup are transmitted to the second special image processing unit 64, and the RGB image signals obtained from illumination using the third illumination light and image pickup are transmitted to the third special image processing unit 65.

The normal image processing unit 62 performs image processing for a normal image on the RGB image signals that are obtained in the normal observation mode. The image processing for a normal image includes structure emphasis processing for a normal image and the like. The RGB image signals having been subjected to the image processing for a normal image are input to the video signal generation unit 66 from the normal image processing unit 62 as a normal image.

The first special image processing unit 63 generates a first special image, which has been subjected to saturation emphasis processing, hue emphasis processing, and structure emphasis processing, on the basis of first RGB image signals (first image) that are obtained in a case where an object to be observed is illuminated with the first illumination light and the image thereof is picked up. Processing to be performed by the first special image processing unit 63 includes processing for emphasizing superficial blood vessels. In the first special image, not only superficial blood vessels are emphasized but also a color difference between a plurality of ranges included in the object to be observed is increased. Further, structures of the plurality of ranges included in the object to be observed are emphasized in the first special image. The details of the first special image processing unit 63 will be described later. The first special image generated by the first special image processing unit 63 is input to the video signal generation unit 66.

The second special image processing unit 64 generates a second special image, which has been subjected to saturation emphasis processing, hue emphasis processing, and structure emphasis processing, on the basis of second RGB image signals (second image) that are obtained in a case where an object to be observed is illuminated with the second illumination light and the image thereof is picked up. The second special image processing unit 64 includes the same processing sections as those of the first special image processing unit 63, but the contents of processing thereof are different from those of the first special image processing unit 63. For example, processing to be performed by the second special image processing unit 64 includes processing for emphasizing deep blood vessels instead of the processing for emphasizing superficial blood vessels. Further, in the second special image, not only deep blood vessels are emphasized but also a color difference between a plurality of ranges included in the object to be observed is increased. Furthermore, structures of the plurality of ranges included in the object to be observed are emphasized in the second special image. The details of the second special image processing unit 64 will be described later. The second special image generated by the second special image processing unit 64 is input to the video signal generation unit 66.

The third special image processing unit 65 generates a third special image, which has been subjected to saturation emphasis processing, hue emphasis processing, and structure emphasis processing, on the basis of third RGB image signals (third image) that are obtained in a case where an object to be observed is illuminated with the third illumination light and the image thereof is picked up. The third special image processing unit 65 includes the same processing sections as those of the first special image processing unit 63, but the contents of processing thereof are different from those of the first special image processing unit 63. For example, processing to be performed by the third special image processing unit 65 includes processing for emphasizing the blood vessels of an intermediate layer positioned between a surface layer and a deep layer instead of the processing for emphasizing superficial blood vessels. Further, a color difference between a plurality of ranges included in the object to be observed is increased in the third special image. Furthermore, structures of the plurality of ranges included in the object to be observed are emphasized in the third special image. The details of the third special image processing unit 65 will be described later. The third special image generated by the third special image processing unit 65 is input to the video signal generation unit 66.

The video signal generation unit 66 converts the normal image, the first special image, the second special image, or the third special image, which is input from the normal image processing unit 62, the first special image processing unit 63, the second special image processing unit 64, or the third special image processing unit 65, into video signals used to display the normal image, the first special image, the second special image, or the third special image as an image that can be displayed by the monitor 18. The monitor 18 displays the normal image, the first special image, the second special image, or the third special image on the basis of the video signals.

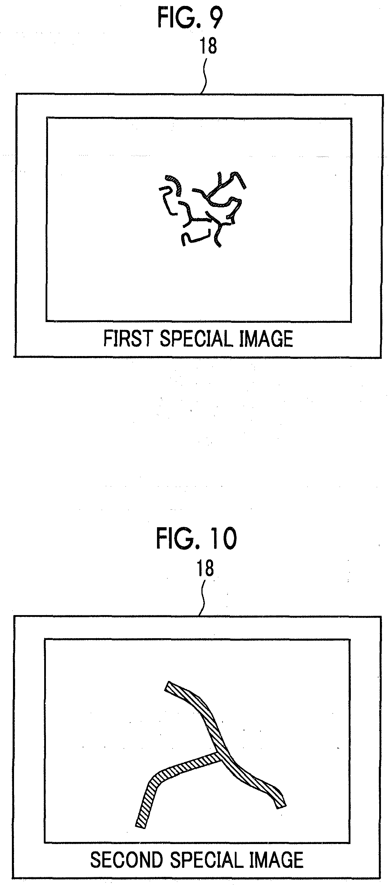

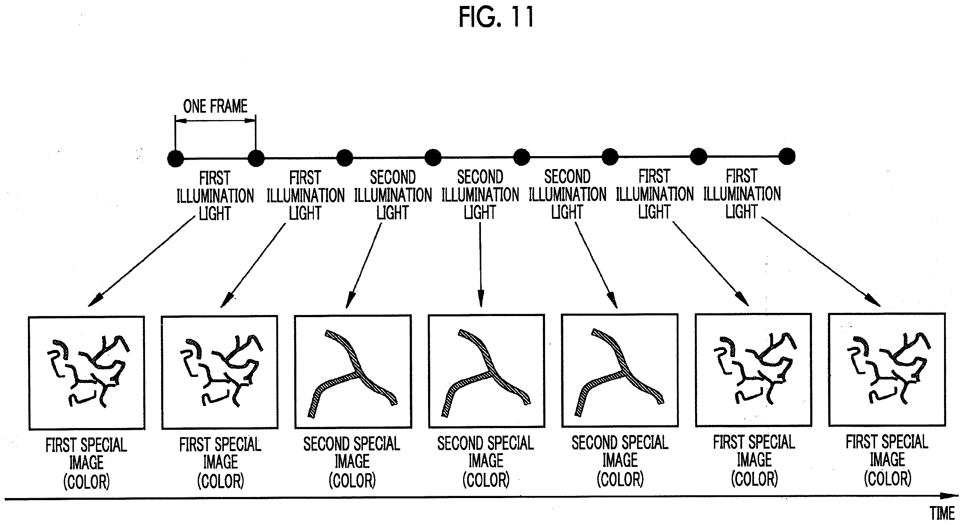

For example, in a case where the first special image is displayed on the monitor 18, superficial blood vessels having a first color are emphasized and displayed in the first special image as shown in Fig. 9. Further, in a case where the second special image is displayed on the monitor 18, deep blood vessels having a second color are emphasized and displayed in the second special image as shown in Fig. 10. It is preferable that the first color and the second color are different from each other. Furthermore, in the multi-observation mode, as shown in Fig. 11, a color first special image and a color second special image are switched and displayed on the monitor 18 according to the light emission period of the first illumination light and the light emission period of the second illumination light. That is, in a case where the light emission period of the first illumination light is two frames and the light emission period of the second illumination light is three frames, the first special image continues to be displayed for two frames and the second special image continues to be displayed for three frames.

As described above, two kinds of the first special image and the second special image are automatically switched and displayed in the multi-observation mode without the operation of the mode changeover SW 13a performed by a user. Further, since the first special image in which superficial blood vessels are emphasized and deep blood vessels are suppressed and the second special image in which deep blood vessels are emphasized and superficial blood vessels are suppressed are switched and displayed, the emphasis and suppression of the two kinds of blood vessels are repeated. Accordingly, the visibility of the plurality of blood vessels having different depths can be improved.

Furthermore, since each of the first special image and the second special image is an image obtained on the basis of illumination light including a red-light wavelength range, a mucous membrane can be reproduced with a tone close to white normal light. Accordingly, since the tone of a mucous membrane in each of the first special image and the second special image displayed in the multi-observation mode is almost not changed from that in the normal image, a sense of incongruity is not given to a user. As a result, a user can learn about the multi-observation mode in a relatively short period. Moreover, since the first special image and the second special image are switched and displayed, it is possible to grasp how blood vessels stand to superficial blood vessels from deep blood vessels.

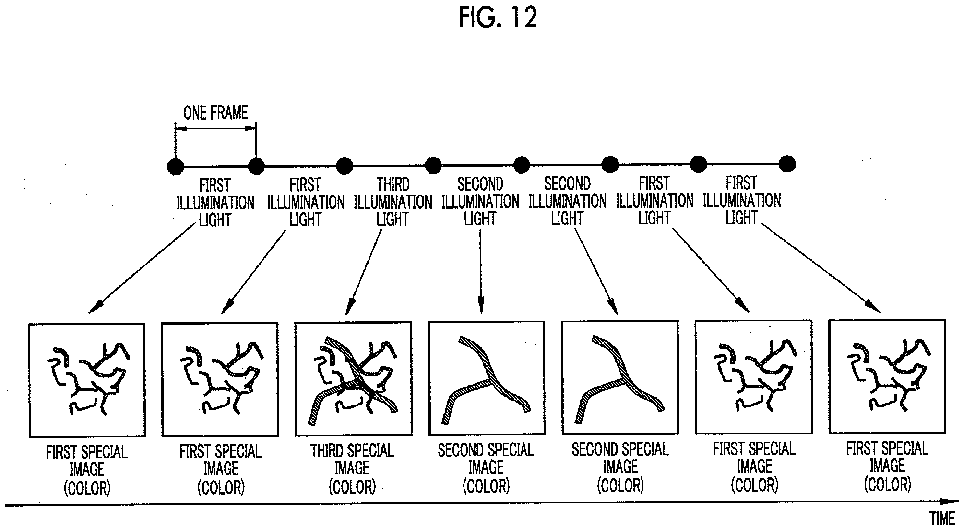

Further, in a case where illumination using the third illumination light is performed at the time of the switching of the first illumination light and the second illumination light in the multi-observation mode, the third special image, which is obtained from the illumination using the third illumination light and image pickup, is displayed on the monitor 18 during the switching of an image to the second special image from the first special image as shown in Fig. 12. The blood vessels of an intermediate layer positioned between a surface layer and a deep layer are displayed in this third special image in addition to both superficial blood vessels and deep blood vessels. Since the third special image is displayed as described above, it is possible to more clearly grasp how blood vessels stand to superficial blood vessels from deep blood vessels. Furthermore, since a change in color becomes gentle by the display of the third special image, a sense of incongruity to be given to a user can be reduced.

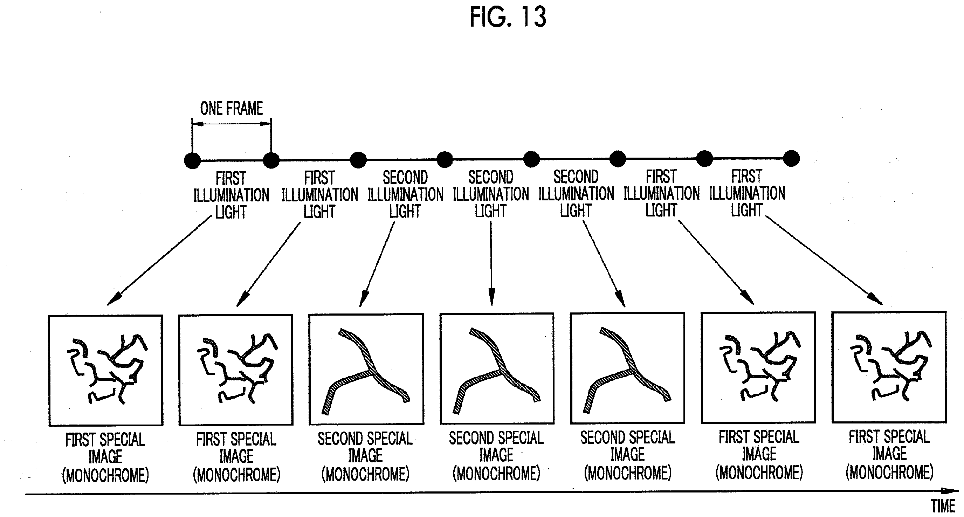

Further, the first special image and the second special image are displayed in the multi-observation mode as color images, but the first special image and the second special image may be displayed as monochrome images instead of color images as shown in Fig. 13. In a case where a monochrome first special image and a monochrome second special image are switched and displayed in this way, a change in color almost does not occur at a portion other than blood vessels, such as superficial blood vessels and deep blood vessels. Accordingly, a user can pay attention to and observe blood vessels having different depths, such as superficial blood vessels or deep blood vessels, without feeling a sense of incongruity at the time of the switching of the first special image and the second special image..

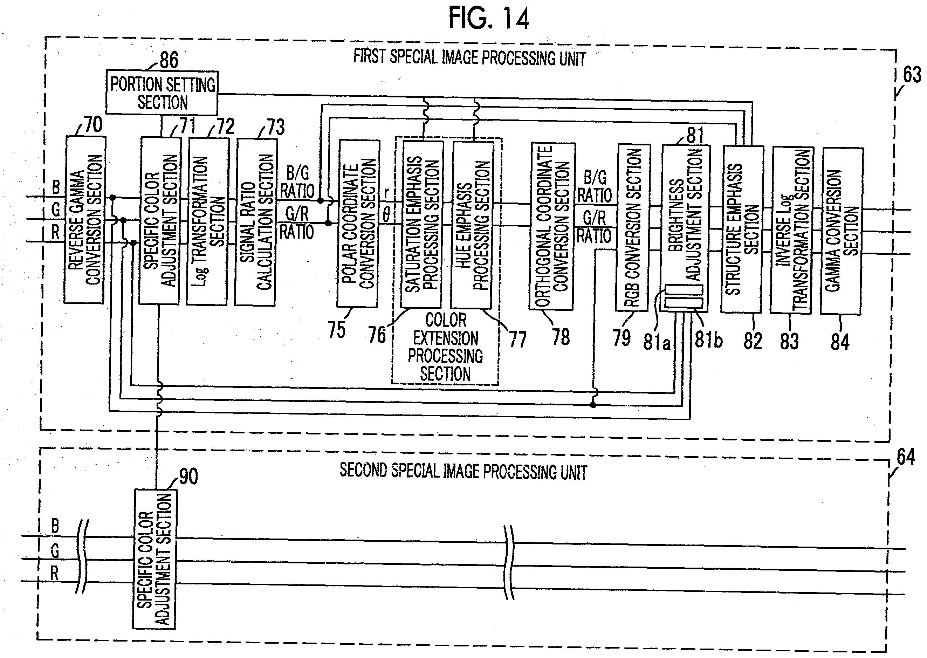

As shown in Fig. 14, the first special image processing unit 63 comprises a reverse gamma conversion section 70, a specific color adjustment section 71, a Log transformation section 72, a signal ratio calculation section 73, a polar coordinate conversion section 75, a saturation emphasis processing section 76, a hue emphasis processing section 77, an orthogonal coordinate conversion section 78, an RGB conversion section 79, a brightness adjustment section 81, a structure emphasis section 82, an inverse Log transformation section 83, and a gamma conversion section 84. Further, the first special image processing unit 63 is provided with a portion setting section 86 that sets a portion, which is being currently observed, to change an adjustment level in the specific color adjustment section 71 or an emphasis level in the saturation emphasis processing section 76, the hue emphasis processing section 77, and the structure emphasis section 82 depending on a portion to be observed, such as the gullet, the stomach, or the large intestine. The portion setting section 86 may set a portion, which is being currently observed, (for example, the gullet, the stomach, or the large intestine) through the console 19 or may set a portion by automatically recognizing the portion from an image obtained during the current observation.

The reverse gamma conversion section 70 performs reverse gamma conversion on the first RGB image signals that are obtained in a case where the object to be observed is illuminated with the first illumination light and the image thereof is picked up. Since the first RGB image signals having been subjected to the reverse gamma conversion are reflectance-linear first RGB signals that are linear in terms of reflectance from a specimen, the percentage of signals, which are related to various kinds of biological information about the specimen, among the first RGB image signals is high. Reflectance-linear first R-image signals are referred to as R1x-image signals, reflectance-linear first G-image signals are referred to as G1x-image signals, and reflectance-linear first B-image signals are referred to as B1x-image signals.

The specific color adjustment section 71 performs first mucous membrane-color-balance processing for automatically adjusting the color of a mucous membrane, which is included in the object to be observed, on the basis of the portion set by the portion setting section 86 and the R1x-image signals, the G1x-image signals, and the B1x-image signals. In the first mucous membrane-color-balance processing, the average colors of the entire screen are automatically adjusted using, for example, D1) to D3) so as to correspond to specific color balance. Since the first mucous membrane-color-balance processing is performed, R1x-image signals, G1x-image signals, and B1x-image signals having been subjected to the first mucous membrane-color-balance processing are obtained.

Equation D1) R1x having been subjected to first mucous membrane-color-balance

processing $= R1x/R1ave \times \alpha\_n$

Equation D2) G1x having been subjected to first mucous membrane-color-balance

processing $= G1x/G1ave \times \beta\_n$

Equation D3) B1x having been subjected to first mucous membrane-color-balance processing $=B1x/B1ave \times \gamma\_n$

However, the first mucous membrane-color-balance processing is processing to be performed on the assumption that the color of a mucous membrane is dominant over the entire object to be observed.

In Equations D1) to D3), R1ave denotes the average pixel value of the R1x-image signals (the sum of the pixel values of the entire screen (effective pixels)/the number of effective pixels). G1ave denotes the average pixel value of the G1x-image signals (the sum of the pixel values of the entire screen (effective pixels)/the number of effective pixels). B1ave denotes the average pixel value of the B1x-image signals (the sum of the pixel values of the entire screen (effective pixels)/the number of effective pixels). Further, $\alpha\_n$ (n=0, 1, 2) denotes a correction factor used to correct the R1x-image signal, $\beta\_n$ (n=0, 1, 2) denotes a correction factor used to correct the G1x-image signal, and $\gamma\_n$ (n=0, 1, 2) denotes a correction factor used to correct the B1x-image signal.

In a case where the gullet is set by the portion setting section 86, $\alpha\_0$, $\beta\_0$, and $\gamma\_0$ that are the correction factors for the gullet are used in the arithmetic operations of Equations D1) to D3). In a case where the arithmetic operation of Equation 1) is performed using the correction factors $\alpha\_0$, $\beta\_0$, and $\gamma\_0$ for the gullet, the color of a mucous membrane is made to match a target color corresponding to the gullet. Further, in a case where the stomach is set by the portion setting section 86, $\alpha\_1$, $\beta\_1$, and $\gamma\_1$ that are the correction factors for the stomach are used in the arithmetic operations of Equations D1) to D3). In a case where the arithmetic operations of Equations D1) to D3) are performed using the correction factors $\alpha\_1$, $\beta\_1$, and $\gamma\_1$ for the stomach, the color of a mucous membrane is made to match a target color corresponding to the stomach. Further, in a case where the large intestine is set by the portion setting section 86, $\alpha\_2$, $\beta\_2$, and $y\_2$ that are the correction factors for the large intestine are used in the arithmetic operations of Equations D1) to D3). In a case where the arithmetic operations of Equations D1) to D3) are performed using the correction factors $\alpha\_2$, $\beta\_2$, and $\gamma\_2$ for the large intestine, the color of a mucous membrane is made to match a target color corresponding to the large intestine.

The specific color adjustment section 71 may be adapted to manually adjust the color of a mucous membrane instead of automatically performing the first mucous membrane-color-balance processing. In this case, the specific color adjustment section 71 displays a mucous membrane color-adjustment menu used to adjust the color of a mucous membrane on the monitor 18, and receives an instruction to adjust the color of a mucous membrane to a target color (color adjustment instruction) from the console 19 (color adjustment instruction-receiving unit). In a case where the specific color adjustment section 71 receives an instruction from the console 19, the specific color adjustment section 71 adjusts the pixel values of the R1x-image signals, the G1x-image signals, and the B1x-image signals so that the color of a mucous membrane matches a target color. For example, a correspondence relationship between the amount of operation to be performed by the console 19 and the amounts of adjustment of the pixel values of the R1x-image signals, the G1x-image signals, and the B1x-image signals used to adjust the color of a mucous membrane to a target color is set in advance.

Further, the specific color adjustment section 71 may be adapted to manually adjust superficial blood vessels or deep blood vessels. In this case, the specific color adjustment section 71 displays a blood vessel color-adjustment menu used to adjust superficial blood vessels or deep blood vessels on the monitor 18, and receives an instruction to adjust superficial blood vessels or deep blood vessels to a target color (color adjustment instruction) from the console 19 (color adjustment instruction-receiving unit). In a case where the specific color adjustment section 71 receives an instruction from the console 19, the specific color adjustment section 71 adjusts the pixel values of the R1x-image signals, the G1x-image signals, and the B1x-image signals so that superficial blood vessels or deep blood vessels match a target color. For example, a correspondence relationship between the amount of operation to be performed by the console 19 and the amounts of adjustment of the pixel values of the R1x-image signals, the G1x-image signals, and the B1x-image signals used to adjust superficial blood vessels or deep blood vessels to a target color is set in advance.

Furthermore, the specific color adjustment section 71 may perform the first mucous membrane-color-balance processing using the results of second mucous membrane-color-balance processing, which is performed by a specific color adjustment section 90 of the second special image processing unit 64, to cause the color of a mucous membrane of the first special image to coincide with the color of a mucous membrane of the second special image. In this case, R2ave, G2ave, and B2ave obtained in the second mucous membrane-color-balance processing are used in the first mucous membrane-color-balance processing instead of R1ave, G1ave, and B1ave as shown in Equations DA1) to DA3).

Equation DA1) R1x having been subjected to first mucous membrane-color-balance processing $=R1x/R2ave \times \alpha\_n$

Equation DB1) G1x having been subjected to first mucous membrane-color-balance processing =G1x/G2ave×β_n

Equation DC1) B1x having been subjected to first mucous membrane-color-balance processing =B1x/B2ave×γ_n

In a case where the second mucous membrane-color-balance processing using R2ave, G2ave, and B2ave is performed as described above, the color of a mucous membrane of the first special image and the color of a mucous membrane of the second special image are made to coincide with each other. Here, "the color of a mucous membrane of the first special image and the color of a mucous membrane of the second special image coincide with each other" means a case where a color difference between the color of a mucous membrane of the first special image and the color of a mucous membrane of the second special image is in a predetermined range in addition to a case where the color of a mucous membrane of the first special image and the color of a mucous membrane of the second special image completely coincide with each other. In a case where the specific color adjustment section 90 is to perform the second mucous membrane-color-balance processing by using the results of the first mucous membrane-color-balance processing, R1ave, G1ave, and B1ave obtained by the specific color adjustment section 71 are transmitted to the specific color adjustment section 90.

The Log transformation section 72 performs Log transformation on each of the R1x-image signals, the G1x-image signals, and the B1x-image signals that have been transmitted through the specific color adjustment section 71. Accordingly, Rx-image signals (logR1x) having been subjected to the Log transformation, G1x-image signals (logG1x) having been subjected to the Log transformation, and B1x-image signals (logB1x) having been subjected to the Log transformation are obtained. The signal ratio calculation section 73 calculates a B/G ratio (an object obtained in a case where "-log" and "1x" are omitted from -log(B1x/G1x) is written as "B/G ratio") by performing difference processing (logG1x-logB1x=logG1x/B1x=-log(B1x/G1x)) on the basis of the G1x-image signals and the B1x-image signals having been subjected to the Log transformation. Further, the signal ratio calculation section 73 calculates a G/R ratio by performing difference processing (logR1x-logG1x=logR1x/G1x=-log(G1x/R1x)) on the basis of the R1x-image signals and the G1x-image signals having been subjected to the Log transformation. Like the B/G ratio, the G/R ratio means an object that is obtained in a case where "-log" and "1x" are omitted from -log(G/R).

The B/G ratio and the G/R ratio are obtained for each pixel from the pixel values of pixels that are present at the same positions in the R1x-image signals, the G1x-image signals, and the B1x-image signals. Further, the B/G ratio and the G/R ratio are obtained for each pixel. Furthermore, the B/G ratio is correlated to a blood vessel depth (a distance between the surface of a mucous membrane and a position where a specific blood vessel is present). Accordingly, in a case where a blood vessel depth is changed, the B/G ratio is changed with a change in a blood vessel depth. Moreover, the G/R ratio is correlated to the amount of blood (hemoglobin index). Accordingly, in a case where the amount of blood is changed, the G/R ratio is changed with a change in the amount of blood.

The polar coordinate conversion section 75 converts each of the B/G ratio and the G/R ratio, which are obtained by the signal ratio calculation section 73, into a radius vector r and an angle θ. In the polar coordinate conversion section 75, the conversion of a ratio into a radius vector r and an angle θ is performed over all the pixels. The saturation emphasis processing section 76 performs saturation emphasis processing for increasing a saturation difference between a plurality of ranges, which are included in an object to be observed, by extending or compressing a radius vector r. The details of the saturation emphasis processing section 76 will be described later. The hue emphasis processing section 77 performs hue emphasis processing for increasing a hue difference between a plurality of ranges by increasing or reducing an angle θ. The details of the hue emphasis processing section 77 will also be described later. The saturation emphasis processing section 76 and the hue emphasis processing section 77 function as a color extension processing section that increases a color difference between a plurality of ranges.

The orthogonal coordinate conversion section 78 converts the radius vector r and the angle θ, which have been subjected to the saturation emphasis processing and the hue emphasis processing, into orthogonal coordinates. Accordingly, the radius vector r and the angle θ are converted into a B/G ratio and a G/R ratio that have been subjected to an increase and a reduction in angle. The RGB conversion section 79 converts the B/G ratio and the G/R ratio, which have been subjected to the saturation emphasis processing and the hue emphasis processing, into R1y-image signals, G1y-image signals, and B1y-image signals by using at least one kind of image signals among the R1x-image signals, the G1x-image signals, and the B1x-image signals. For example, the RGB conversion section 79 converts the B/G ratio into the B1y-image signals by performing an arithmetic operation based on the Gx-image signals among the R1x-image signals, the G1x-image signals, and the B1x-image signals and the B/G ratio. Further, the RGB conversion section 79 converts the G/R ratio into the R1y-image signals by performing an arithmetic operation based on the G1x-image signals of the first RGB image signals and the G/R ratio. Furthermore, the RGB conversion section 79 outputs the G1x-image signals as the G1y-image signals without performing special conversion.

The brightness adjustment section 81 adjusts the pixel values of the R1y-image signals, the G1y-image signals, and the B1y-image signals by using the R1x-image signals, the G1x-image signals, and the B1x-image signals and the R1y-image signals, the G1y-image signals, and the B1y-image signals. The reason to adjust the pixel values of the R1y-image signals, the G1y-image signals, and the B1y-image signals by the brightness adjustment section 81 is as follows. There is a possibility that the R1y-image signals, the G1y-image signals, and the B1y-image signals obtained from processing for extending or reducing a color region by the saturation emphasis processing section 76 and the hue emphasis processing section 77 may be significantly changed from the R1x-image signals, the G1x-image signals, and the B1x-image signals in terms of brightness. Accordingly, the pixel values of the R1y-image signals, the G1y-image signals, and the B1y-image signals are adjusted by the brightness adjustment section 81 so that R1y-image signals, G1y-image signals, and B1y-image signals having been subjected to brightness adjustment have the same brightness as the R1x-image signals, the G1x-image signals, and the B1x-image signals.

The brightness adjustment section 81 comprises: a first brightness-information calculation section 81a that obtains first brightness information Yin on the basis of the R1x-image signals, the G1x-image signals, and the B1x-image signals; and a second brightness-information calculation section 81b that obtains second brightness information Yout on the basis of the R1y-image signals, the G1y-image signals, and the B1y-image signals. The first brightness-information calculation section 81a calculates the first brightness information Yin according to an arithmetic expression of "kr×pixel values of R1x-image signals+kgxpixel values of G1x-image signals+kbxpixel values of B1x-image signals". Like the first brightness-information calculation section 81a, the second brightness-information calculation section 81b also calculates the second brightness information Yout according to the same arithmetic expression as described above. In a case where the first brightness information Yin and the second brightness information Yout are obtained, the brightness adjustment section 81 adjusts the pixel values of the R1y-image signals, the G1y-image signals, and the B1y-image signals by performing arithmetic operations based on Equations E1) to E3).

$$\text{E1): } R1y^* = \text{pixel values of R1y-image signals} \times Yin/Yout$$

$$\text{E2): } G1y^* = \text{pixel values of G1y-image signals} \times Yin/Yout$$

$$\text{E3): } B1y^* = \text{pixel values of B1y-image signals} \times Yin/Yout$$

"R1y*" denotes the R1y-image signals having been subjected to brightness adjustment, "G1y*" denotes the G1y-image signals having been subjected to brightness adjustment, and "B1y*" denotes the B1y-image signals having been subjected to brightness adjustment. Further, "kr", "kg", and "kb" are any constants that are in the range of "0" to "1".

The structure emphasis section 82 performs structure emphasis processing on the R1y-image signals, the G1y-image signals, and the B1y-image signals that have been transmitted through the brightness adjustment section 81. The details of the structure emphasis section 82 will be described later. The inverse Log transformation section 83 performs inverse Log transformation on the R1y-image signals, the G1y-image signals, and the B1y-image signals that have been transmitted through the structure emphasis section 82. Accordingly, the R1y-image signals, the G1y-image signals, and the B1y-image signals having pixel values of antilogarithms are obtained. The gamma conversion section 84 performs gamma conversion on the image signals that have been transmitted through the inverse Log transformation section 83. Accordingly, the R1y-image signals, the G1y-image signals, and the B1y-image signals having gradations suitable for an output device, such as the monitor 18, are obtained. The R1y-image signals, the G1y-image signals, and the B1y-image signals having been transmitted through the gamma conversion section 84 are sent to the video signal generation unit 66.

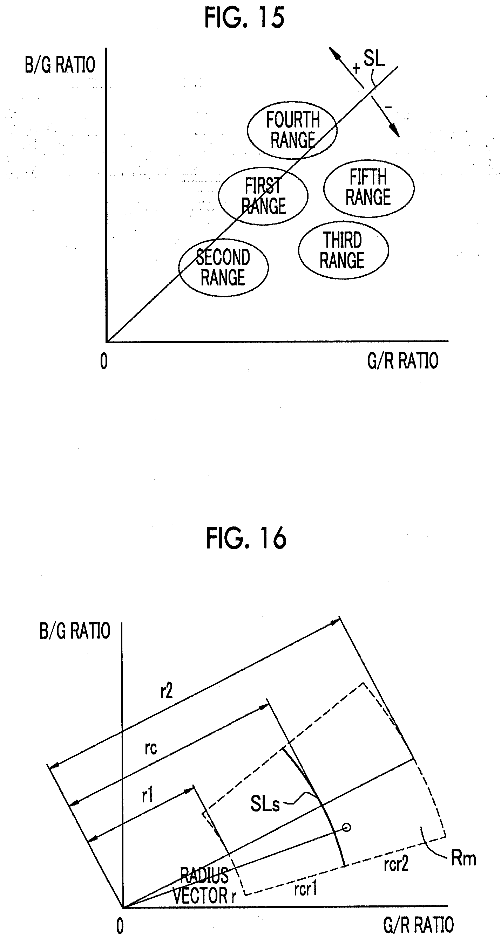

As shown in Fig. 15, the saturation emphasis processing section 76 and the hue emphasis processing section 77 increase a saturation difference or a hue difference between a first range including a normal mucous membrane, a second range including an atrophic mucous membrane, a third range including deep blood vessels present below the atrophic mucous membrane (hereinafter, simply referred to as deep blood vessels), a fourth range including a brownish area (BA), and a fifth range including redness, as a plurality of ranges included in an object to be observed. The first range including a normal mucous membrane is distributed substantially in the center of the first quadrant of a signal ratio space (feature space) formed from the B/G ratio and the G/R ratio. The second range including an atrophic mucous membrane is positioned substantially on the clockwise side (the negative side to be described later) of a reference line SL passing through the first range including a normal mucous membrane, and is distributed at a position that is closer to the origin than the first range including a normal mucous membrane.

The third range including deep blood vessels is distributed on the clockwise side (the negative side to be described later) of the reference line SL. The fourth range including a BA is distributed substantially on the counterclockwise side (the positive side to be described later) of the reference line SL. The fifth range including redness is distributed on the clockwise side (the negative side to be described later) of the reference line SL. The fourth range including a BA and the fifth range including redness are distributed at positions that are farther from the origin than the first range including a normal mucous membrane. It is preferable that a normal mucous membrane is included in a normal portion of an object to be observed; and an atrophic mucous membrane, deep blood vessels, a BA, and redness are included in an abnormal portion of the object to be observed. Further, the reference line SL corresponds to a hue reference line SLh to be described later.

As shown in Fig. 16, the saturation emphasis processing section 76 changes a radius vector r that is represented by coordinates positioned within a radius vector change range Rm in the signal ratio space and does not change a radius vector r that is represented by coordinates positioned outside the radius vector change range Rx. The radius vector change range Rm is a range where a radius vector r is in the range of "r1" to "r2" (r1<r2). Further, a saturation reference line SLs is set on a radius vector rc between a radius vector r1 and a radius vector r2 in the radius vector change range Rm. Here, as the radius vector r is larger, a saturation is higher. Accordingly, a range rcr1 (r1<r<rc) where the radius vector r is smaller than the radius vector rc indicated by the saturation reference line SLs is referred to as a low-saturation range. On the other hand, a range rcr2 (rc<r<r2) where the radius vector r is larger than the radius vector rc indicated by the saturation reference line SLs is referred to as a high-saturation range.

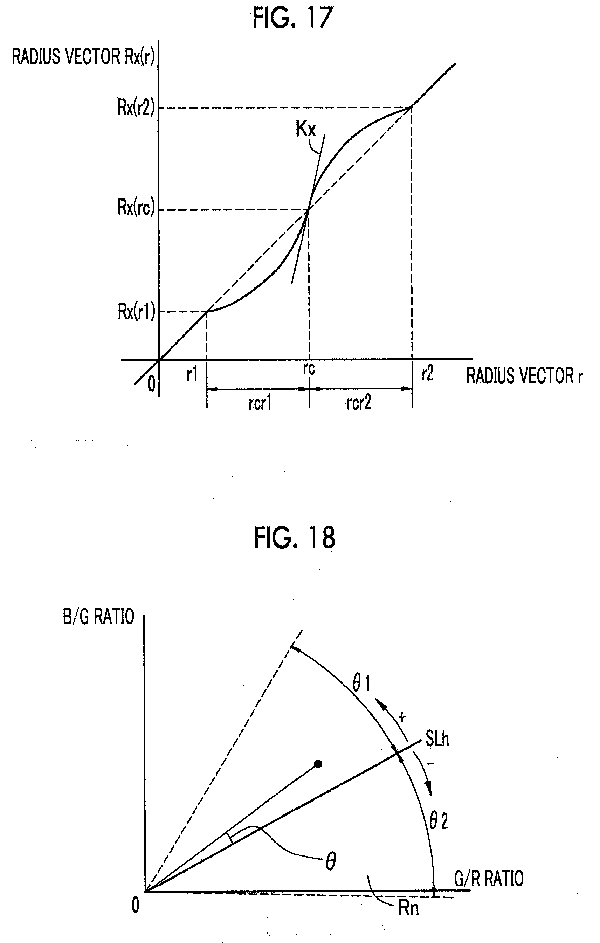

In the saturation emphasis processing performed by the saturation emphasis processing section 76, as shown in Fig. 17, a radius vector Rx(r) is output in a case where the radius vector r of the coordinates included in the radius vector change range Rm is input. A relationship between an input and an output according to this saturation emphasis processing is shown by a solid line. The saturation emphasis processing causes the output Rx(r) to be smaller than the input r in the low-saturation range rcr1, and causes the output Rx(r) to be larger than the input r in the high-saturation range rcr2. Further, an inclination Kx at Rx(rc) is set to "1" or more. Accordingly, it is possible to further reduce the saturation of an object to be observed included in the low-saturation range and to further increase the saturation of an object to be observed included in the high-saturation range. A saturation difference between a plurality of ranges can be increased by the emphasis of a saturation.

Since the color of a mucous membrane of an object to be observed varies depending on a portion, it is preferable that the result of the saturation emphasis processing as one of color extension processing is adjusted using an adjustment parameter determined for each portion. For example, the saturation of Rx(r) output from the saturation emphasis processing section 76 is adjusted using an adjustment parameter determined for each portion. In a case where the gullet is set by the portion setting section 86, Rx(r) is multiplied by an adjustment parameter P0 for the gullet to adjust a saturation. Further, in a case where the stomach is set by the portion setting section 86, Rx(r) is multiplied by an adjustment parameter P1 for the stomach to adjust a saturation. Furthermore, in a case where the large intestine is set by the portion setting section 86, Rx(r) is multiplied by an adjustment parameter P2 for the large intestine to adjust a saturation.

As shown in Fig. 18, the hue emphasis processing section 77 changes an angle $\theta$ that is represented by coordinates positioned within an angle change range Rn in the signal ratio space and does not change an angle $\theta$ that is represented by coordinates positioned outside the angle change range Rn. The angle change range Rn includes the range of an angle $\theta 1$ from the hue reference line SLh in a counterclockwise direction (positive direction) and the range of an angle $\theta 2$ from the hue reference line SLh in a clockwise direction (negative direction). The angle $\theta$ of the coordinates included in the angle change range Rn is redefined by an angle $\theta$ from the hue reference line SLh. In a case where the angle $\theta$ is changed, a hue is also changed. Accordingly, the range of the angle $\theta 1$ of the angle change range Rn is referred to as a positive-side hue range $\theta 1$ and the range of the angle $\theta 2$ thereof is referred to as a negative-side hue range $\theta 2$.

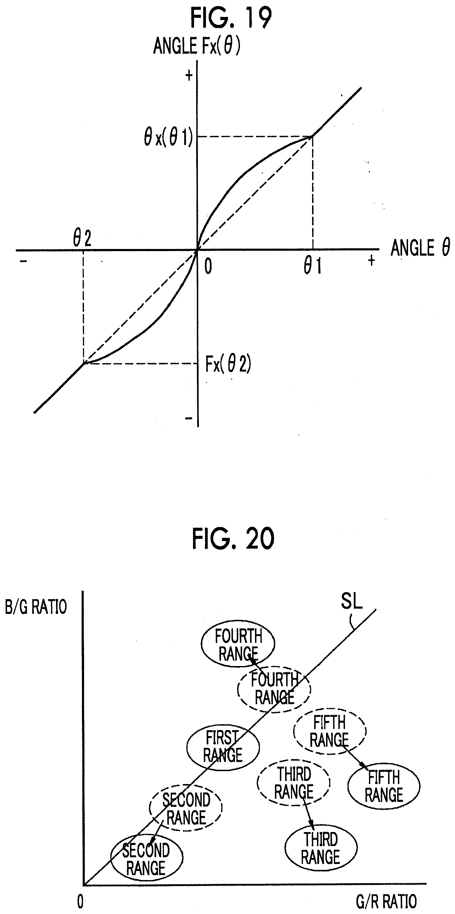

In the hue emphasis processing performed by the hue emphasis processing section 77, as shown in Fig. 19, an angle Fx($\theta$) is output in a case where the angle $\theta$ of the coordinates included in the angle change range Rn is input. A relationship between an input and an output according to the first hue emphasis processing is shown by a solid line. The hue emphasis processing causes the output Fx($\theta$) to be smaller than the input $\theta$ in the negative-side hue range $\theta 2$, and causes the output Fx($\theta$) to be larger than the input $\theta$ in the positive-side hue range $\theta 1$. Accordingly, it is possible to increase a hue difference between an object to be observed included in the negative-side hue range and an object to be observed included in the positive-side hue range. A hue difference between a plurality of ranges can be increased by the emphasis of a hue.

Since the color of a mucous membrane of an object to be observed varies depending on a portion, it is preferable that the result of the hue emphasis processing as one of color extension processing is adjusted using an adjustment parameter determined for each portion. For example, the hue of Fx($\theta$) output from the hue emphasis processing section 77 is adjusted using an adjustment parameter corresponding to each portion. In a case where the gullet is set by the portion setting section 86, Fx(θ) is multiplied by an adjustment parameter Q0 for the gullet to adjust a hue. Further, in a case where the stomach is set by the portion setting section 86, Fx(r) is multiplied by an adjustment parameter Q1 for the stomach to adjust a hue. Furthermore, in a case where the large intestine is set by the portion setting section 86, Fx(θ) is multiplied by an adjustment parameter Q2 for the large intestine to adjust a hue.

Since the saturation emphasis processing and the hue emphasis processing are performed as described above, a difference between the first range including a normal mucous membrane and the second range (solid line) including an atrophic mucous membrane having been subjected to the saturation emphasis processing and the hue emphasis processing is larger than a difference between the first range including a normal mucous membrane and the second range (dotted line) including an atrophic mucous membrane having not yet been subjected to the saturation emphasis processing and the hue emphasis processing as shown in Fig. 20. Likewise, a difference between the first range including a normal mucous membrane and each of deep blood vessels (solid line), the fourth range (solid line) including a BA, and the fifth range (solid line) including redness having been subjected to the saturation emphasis processing and the hue emphasis processing is larger than a difference between the first range including a normal mucous membrane and each of deep blood vessels (dotted line), the fourth range (dotted line) including a BA, and the fifth range (dotted line) including redness having not yet been subjected to the saturation emphasis processing and the hue emphasis processing.

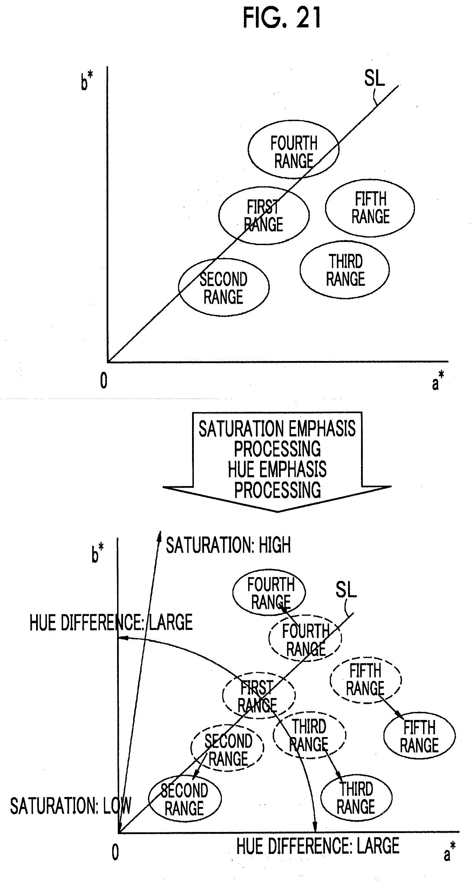

As in the signal ratio space, the first range including a normal mucous membrane, the second range including an atrophic mucous membrane, the third range including deep blood vessels, the fourth range including a BA, and the fifth range including redness are distributed as shown in Fig. 21 even in a feature space (ab space) formed from a* and b* (denoting tint elements a* and b* of a CIE Lab space that is color information. The same hereinafter) that are obtained in a case where the Rlx-image signals, the Glx-image signals, and the B1x-image signals are subjected to Lab conversion by a Lab conversion section. Then, saturation emphasis processing for extending or compressing a radius vector r is performed and hue emphasis processing for increasing or reducing an angle θ are performed in the same method as the above-mentioned method. Since the hue emphasis processing and the saturation emphasis processing are performed, a difference between the first range including a normal mucous membrane and each of the second range (solid line) including an atrophic mucous membrane, the third range (solid line) including deep blood vessels, the fourth range (solid line) including a BA, and the fifth range (solid line) including redness having been subjected to the saturation emphasis processing and the hue emphasis processing is larger than a difference between the first range including a normal mucous membrane and each of the second range (dotted line) including an atrophic mucous membrane, the third range (dotted line) including deep blood vessels, the fourth range (dotted line) including a BA, and the fifth range (dotted line) including redness having not yet been subjected to the saturation emphasis processing and the hue emphasis processing.

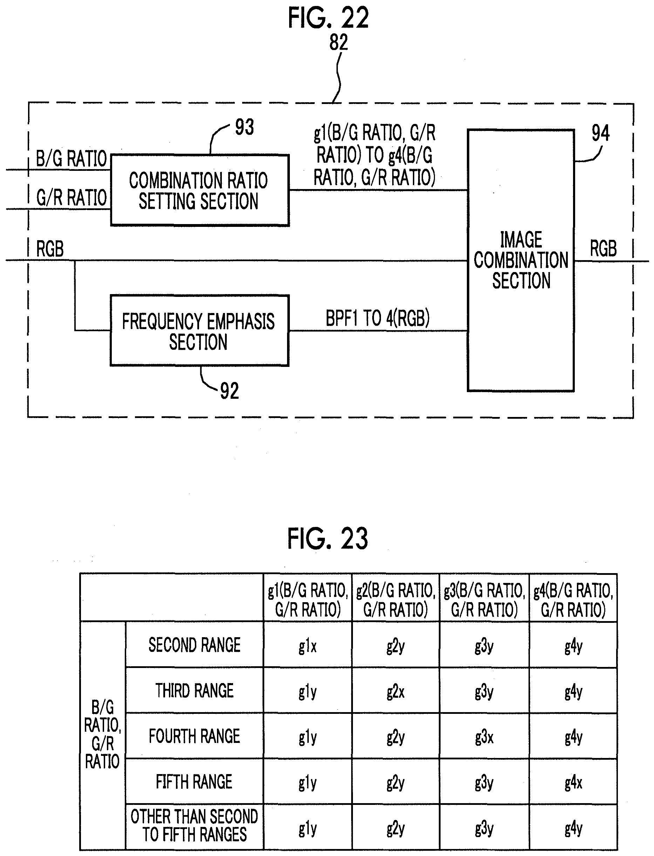

As shown in Fig. 22, the structure emphasis section 82 performs structure emphasis for a specific range, which is included in an object to be observed, as the structure emphasis processing. The specific range to be subjected to structure emphasis includes the second range including an atrophic mucous membrane, the third range including deep blood vessels, the fourth range including a BA, or the fifth range including redness. The structure emphasis section 82 comprises a frequency emphasis section 92, a combination ratio setting section 93, and an image combination section 94. The frequency emphasis section 92 obtains a plurality of frequency-emphasized images by performing plural kinds of frequency filtering (band pass filtering (BPF)) on each of the R1y-image signals, the Gly-image signals, and the B1y-image signals. It is preferable that the structure emphasis section 82 performs processing for emphasizing superficial blood vessels.

The frequency emphasis section 92 uses frequency filtering for an atrophic-mucous-membrane region that extracts low-frequency first frequency components including many atrophic-mucous-membrane regions, frequency filtering for a deep-blood-vessel region that extracts intermediate-frequency second frequency components including many deep-blood-vessel regions, frequency filtering for a BA that extracts low-frequency third frequency components including many BA regions, and frequency filtering for redness that extracts low-frequency fourth frequency components including many reddish regions.

In a case where the frequency filtering for an atrophic-mucous-membrane region is performed, a first frequency component-emphasized image BPF1(RGB) is obtained. BPF1(RGB) represents image signals where the frequency filtering for an atrophic-mucous-membrane region is performed on each of the R1y-image signals, the G1y-image signals, and the B1y-image signals. In a case where the frequency filtering for a deep-blood-vessel region is performed, a second frequency component-emphasized image BPF2(RGB) is obtained. BPF2(RGB) represents image signals where the frequency filtering for a deep-blood-vessel region is performed on each of the R1y-image signals, the G1y-image signals, and the B1y-image signals.

In a case where the frequency filtering for a BA region is performed, a third frequency component-emphasized image BPF3(RGB) is obtained. BPF3 (RGB) represents image signals where the frequency filtering for a BA region is performed on each of the R1y-image signals, the G1y-image signals, and the B1y-image signals. In a case where the frequency filtering for a reddish region is performed, a fourth frequency component-emphasized image BPF4 (RGB) is obtained. BPF4 (RGB) represents image signals where the frequency filtering for a reddish region is performed on each of the R1y-image signals, the G1y-image signals, and the B1y-image signals.

The combination ratio setting section 93 sets combination ratios g1(B/G ratio, G/R ratio), g2(B/G ratio, G/R ratio), g3(B/G ratio, G/R ratio), and g4(B/G ratio, G/R ratio), which represent the combination ratios of first to fourth frequency component-emphasized images BPF1(RGB) to BPF4(RGB) with respect to the R1y-image signals, the G1y-image signals, and the B1y-image signals, for every pixel on the basis of the B/G ratio and G/R ratio (see Fig. 14) having not yet been subjected to the saturation emphasis processing and the hue emphasis processing.

As shown in Fig. 23, the combination ratio g1(B/G ratio, G/R ratio) of a pixel of which the B/G ratio and the G/R ratio are in the second range is set to g1x and the combination ratios g1(B/G ratio, G/R ratio) of pixels of which the B/G ratios and the G/R ratios are in the other ranges (the third range, the fourth range, and the fifth range) are set to g1y. g1x is set to be large to add a first frequency component image to a pixel of which the B/G ratio and the G/R ratio are in the second range. For example, g1x is "100%". In contrast, g1y is set to be extremely small not to add or almost not to add a first frequency component image. For example, g1y is "0%".

The combination ratio g2(B/G ratio, G/R ratio) of a pixel of which the B/G ratio and the G/R ratio are in the third range is set to g2x and the combination ratios g2(B/G ratio, G/R ratio) of pixels of which the B/G ratios and the G/R ratios are in the other ranges (the second range, the fourth range, and the fifth range) are set to g2y. g2x is set to be large to add a second frequency component image to a pixel of which the B/G ratio and the G/R ratio are in the third range. For example, g2x is "100%". In contrast, g2y is set to be extremely small not to add or almost not to add a second frequency component image. For example, g2y is "0%".

The combination ratio g3(B/G ratio, G/R ratio) of a pixel of which the B/G ratio and the G/R ratio are in the fourth range is set to g3x and the combination ratios g3(B/G ratio, G/R ratio) of pixels of which the B/G ratios and the G/R ratios are in the other ranges (the second range, the third range, and the fifth range) are set to g3y. g3x is set to be large to add a third frequency component image to a pixel of which the B/G ratio and the G/R ratio are in the fourth range. For example, g3x is "100%". In contrast, g3y is set to be extremely small not to add or almost not to add a third frequency component image. For example, g3y is "0%".

The combination ratio g4(B/G ratio, G/R ratio) of a pixel of which the B/G ratio and the G/R ratio are in the fifth range is set to g4x and the combination ratios g4(B/G ratio, G/R ratio) of pixels of which the B/G ratios and the G/R ratios are in the other ranges (the second range, the third range, and the fourth range) are set to g4y. g4x is set to be large to add a fourth frequency component image to a pixel of which the B/G ratio and the G/R ratio are in the fifth range. For example, g4x is "100%". In contrast, g4y is set to be extremely small not to add or almost not to add a fourth frequency component image. For example, g4y is "0%".

The image combination section 94 combines the R1y-image signals, the G1y-image signals, and the B1y-image signals with the first to fourth frequency component-emphasized images BPF1(RGB) to BPF4(RGB) on the basis of Equation F) according to the combination ratios that are set for every pixel by the combination ratio setting section 93. Accordingly, R1y-image signals, G1y-image signals, and B1y-image signals having been subjected to the structure emphasis processing are obtained.

Equation F): R1y-image signals, G1y-image signals, and B1y-image signals having been subjected to structure emphasis processing=R1y-image signals, G1y-image signals, and B1y-image signals+BPF1(RGB)×Gain1(RGB)×g1(B/G ratio, G/R ratio)+BPF2(RGB)×Gain2(RGB)×g2(B/G ratio, G/R ratio)+BPF3(RGB)×Gain3(RGB)×g3(B/G ratio, G/R ratio)+BPF4(RGB)×Gain4(RGB)×g4(B/G ratio, G/R ratio)

Gain1(RGB) to Gain4(RGB) of Equation F) are determined in advance according to the edge characteristics of the first to fourth frequency component-emphasized images. For example, since deep blood vessels and a BA form down edges of which the values of images are smaller than "0" in the second and third frequency component-emphasized images including many deep blood vessels and many BAs, it is preferable that Gain2(RGB) and Gain3(RGB) are set to negative values.

Here, the combination ratio g1(B/G ratio, G/R ratio) of a pixel of which the B/G ratio and the G/R ratio are in the second range is set to g1x and the combination ratios g2(B/G ratio, G/R ratio), g3(B/G ratio, G/R ratio), and g4(B/G ratio, G/R ratio) of the pixel are set to g2y, g3y, and g4y by the combination ratio setting section 93. Accordingly, a first frequency emphasis component is added to the pixel of which the B/G ratio and the G/R ratio are in the second range, and a first frequency emphasis component is almost not or never added to pixels of which the B/G ratios and the G/R ratios are in the third to fifth ranges. Since many atrophic-mucous-membrane regions are included in the second range, an atrophic mucous membrane can be subjected to structure emphasis by the addition of a first frequency emphasis component.

Further, the combination ratio g2(B/G ratio, G/R ratio) of a pixel of which the B/G ratio and the G/R ratio are in the third range is set to g2x and the combination ratios g1(B/G ratio, G/R ratio), g3(B/G ratio, G/R ratio), and g4(B/G ratio, G/R ratio) of the pixel are set to g1y, g3y, and g4y by the combination ratio setting section 93. Accordingly, a second frequency emphasis component is added to the pixel of which the B/G ratio and the G/R ratio are in the third range, and a second frequency emphasis component is almost not or never added to pixels of which the B/G ratios and the G/R ratios are in the second, fourth, and fifth ranges. Since many deep-blood-vessel regions are included in the third range, the deep-blood-vessel regions can be subjected to structure emphasis by the addition of a second frequency emphasis component.

Furthermore, the combination ratio g3(B/G ratio, G/R ratio) of a pixel of which the B/G ratio and the G/R ratio are in the fourth range is set to g3x and the combination ratios g1(B/G ratio, G/R ratio), g2(B/G ratio, G/R ratio), and g4(B/G ratio, G/R ratio) of the pixel are set to g1y, g2y, and g4y by the combination ratio setting section 93. Accordingly, a third frequency emphasis component is added to the pixel of which the B/G ratio and the G/R ratio are in the fourth range, and a third frequency emphasis component is almost not or never added to pixels of which the B/G ratios and the G/R ratios are in the second, third, and fifth ranges. Since many BA regions are included in the fourth range, the BA regions can be subjected to structure emphasis by the addition of a third frequency emphasis component.

Moreover, the combination ratio g4(B/G ratio, G/R ratio) of a pixel of which the B/G ratio and the G/R ratio are in the fifth range is set to g4x and the combination ratios g1(B/G ratio, G/R ratio), g2(B/G ratio, G/R ratio), and g3(B/G ratio, G/R ratio) of the pixel are set to g1y, g2y, and g3y by the combination ratio setting section 93. Accordingly, a fourth frequency emphasis component is added to the pixel of which the B/G ratio and the G/R ratio are in the fifth range, and a fourth frequency emphasis component is almost not or never added to pixels of which the B/G ratios and the G/R ratios are in the second to fourth ranges. Since many reddish regions are included in the fifth range, the reddish regions can be subjected to structure emphasis by the addition of a fourth frequency emphasis component.

As described above, the combination ratios are set for every pixel on the basis of the B/G ratio and the G/R ratio, and frequency component-emphasized images are combined with the R1y-image signals, the G1y-image signals, and the B1y-image signals on the basis of the combination ratios that are set for every pixel. Accordingly, specific ranges, such as the atrophic-mucous-membrane regions, the deep-blood-vessel regions, a BA, and redness, can be selectively emphasized. For example, in a case where the first or third frequency component-emphasized image is added to all pixels of a color difference-emphasized image irrespective of the B/G ratio and the G/R ratio, both of the atrophic mucous membrane and a BA are emphasized since the first or third frequency component image is an image of which the low-frequency component is emphasized. Accordingly, in a case where the first frequency component-emphasized image is added to only pixels, of which the B/G ratios and the G/R ratios are in the second range, of a color difference-emphasized image as in the invention, only an atrophic mucous membrane can be emphasized without the emphasis of a BA. In contrast, in a case where the third frequency component-emphasized image is added to only pixels, of which the B/G ratios and the G/R ratios are in the fourth range, of a color difference-emphasized image, only an atrophic mucous membrane can be emphasized without the emphasis of a BA.

Since the color of a mucous membrane of an object to be observed varies depending on a portion, it is preferable that the pixel values of the R1y-image signals, the G1y-image signals, and the B1y-image signals having been subjected to the structure emphasis processing are adjusted using adjustment parameters corresponding to portions. For example, in a case where the gullet is set by the portion setting section 86, the R1y-image signals, the G1y-image signals, and the B1y-image signals having been subjected to the structure emphasis processing are multiplied by an adjustment parameter S0 for the gullet to adjust pixel values. Further, in a case where the stomach is set by the portion setting section 86, the R1y-image signals, the G1y-image signals, and the B1y-image signals having been subjected to the structure emphasis processing are multiplied by an adjustment parameter S1 for the stomach to adjust pixel values. Furthermore, in a case where the large intestine is set by the portion setting section 86, the R1y-image signals, the G1y-image signals, and the B1y-image signals having been subjected to the structure emphasis processing are multiplied by an adjustment parameter S2 for the large intestine to adjust pixel values.

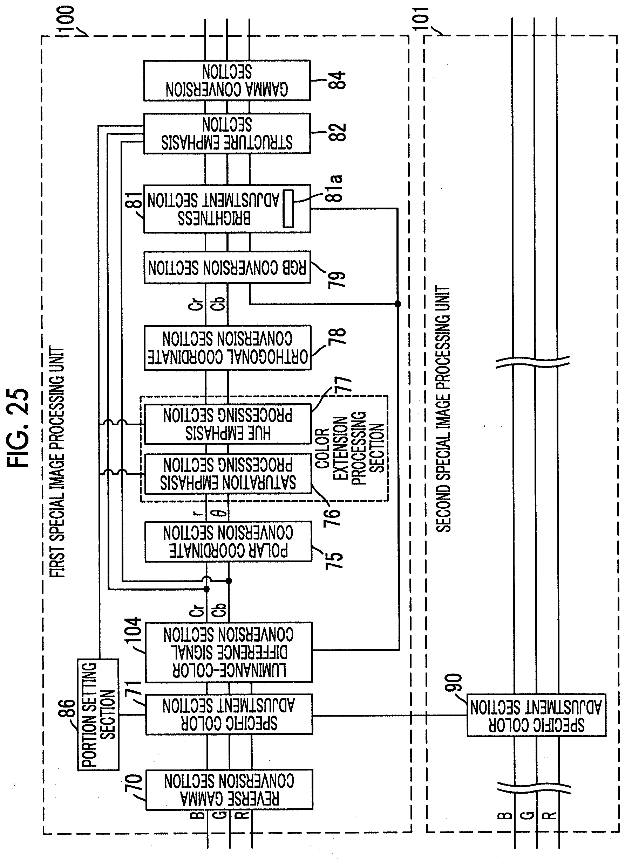

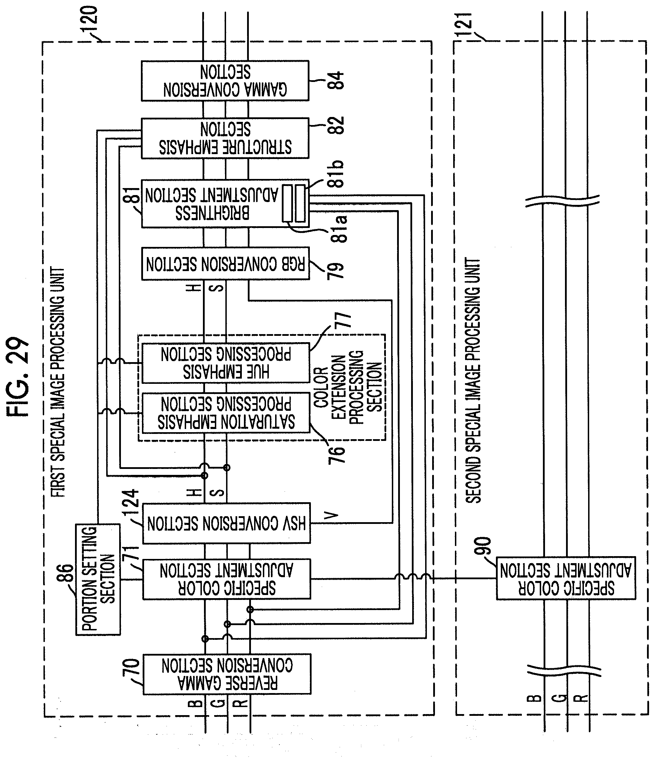

As shown in Fig. 14, the second special image processing unit 64 includes the same processing sections as those of the first special image processing unit 63. However, the contents of processing of the second special image processing unit 64 are partially different from those of the first special image processing unit 63. For example, it is preferable that the structure emphasis section 82 of the second special image processing unit 64 performs processing for emphasizing deep blood vessels. Further, among second RGB image signals having been subjected to reverse gamma conversion by the second special image processing unit 64, reflectance-linear second R-image signals are referred to as R2x-image signals, reflectance-linear second G-image signals are referred to as G2x-image signals, and reflectance-linear second B-image signals are referred to as B2x-image signals. Although not shown in Fig. 14 (the same applies to Figs. 25 and 29), the third special image processing unit 65 includes the same processing sections as those of the first special image processing unit 63. The contents of processing of the third special image processing unit 65 are partially different from those of the first special image processing unit 63. For example, it is preferable that the structure emphasis section 82 of the third special image processing unit 65 performs processing for emphasizing the blood vessels of an intermediate layer positioned between a surface layer and a deep layer.

The specific color adjustment section 90 of the second special image processing unit 64 performs second mucous membrane-color-balance processing for automatically adjusting the color of a mucous membrane included in an object to be observed on the basis of the portion set by the portion setting section 86 and the R2x-image signals, the G2x-image signals, and the B2x-image signals. The second mucous membrane-color-balance processing is the same as the first mucous membrane-color-balance processing, and is performed using, for example, G1) to G3). Accordingly, R2x-image signals, G2x-image signals, and B2x-image signals having been subjected to the second mucous membrane-color-balance processing are obtained.

Equation G1) R2x having been subjected to second mucous membrane-color-balance processing =R2x/R2ave×α_n

Equation G2) G2x having been subjected to second mucous membrane-color-balance processing =G2x/G2ave×β_n

Equation G3) B2x having been subjected to second mucous membrane-color-balance processing =B2x/B2ave×γ_n

However, like the first mucous membrane-color-balance processing, the second mucous membrane-color-balance processing is processing to be performed on the assumption that the color of a mucous membrane is dominant over the entire object to be observed.

In Equations G1) to G3), R2ave denotes the average pixel value of the R2x-image signals (the sum of the pixel values of the entire screen (effective pixels)/the number of effective pixels). G2ave denotes the average pixel value of the G2x-image signals (the sum of the pixel values of the entire screen (effective pixels)/the number of effective pixels). B2ave denotes the average pixel value of the B2x-image signals (the sum of the pixel values of the entire screen (effective pixels)/the number of effective pixels). Further, α_n (n=0, 1, 2), β_n (n=0, 1, 2), and γ_n (n=0, 1, 2) denote correction factors that are used to correct the R2x-image signals, the G2x-image signals, and the B2x-image signals, respectively.

Like the specific color adjustment section 71, the specific color adjustment section 90 may be adapted to manually adjust the color of a mucous membrane, the color of superficial blood vessels, or the color of deep blood vessels instead of automatically performing the second mucous membrane-color-balance processing. The manual adjustment of the color of a mucous membrane is the same as that in the case of the specific color adjustment section 71.

Further, the specific color adjustment section 90 may perform the second mucous membrane-color-balance processing using the results of the first mucous membrane-color-balance processing, which is performed by the specific color adjustment section 71 of the first special image processing unit 63, to cause the color of a mucous membrane of the first special image to coincide with the color of a mucous membrane of the second special image. A method of performing the second mucous membrane-color-balance processing using the results of the first mucous membrane-color-balance processing is the same as the method of performing the first mucous membrane-color-balance processing using the results of the second mucous membrane-color-balance processing as described above.

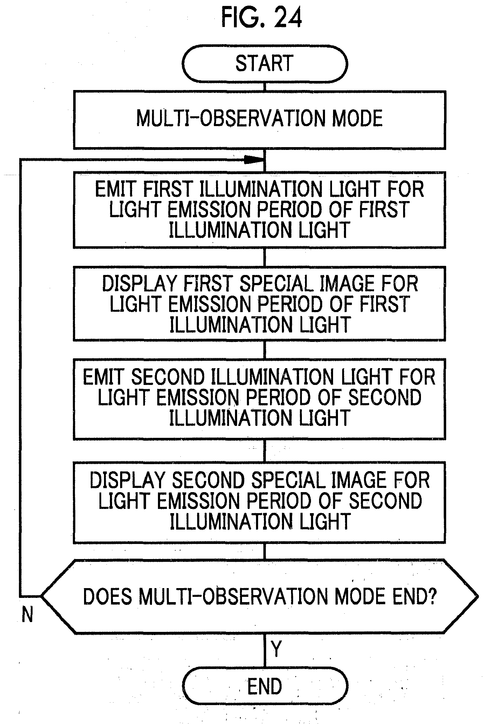

Next, a multi-observation mode will be described with reference to a flowchart of Fig. 24. A mode is switched to the multi-observation mode by the operation of the mode changeover SW 13a. In a case where a mode is switched to the multi-observation mode, the first illumination light continues to be emitted for only the light emission period of the first illumination light that is set in advance. For example, in a case where the light emission period of the first illumination light is two frames, the first illumination light continues to be emitted for two frames. The first special image obtained from the image pickup of an object to be observed, which is being illuminated with the first illumination light, continues to be displayed on the monitor 18 for only the light emission period of the first illumination light.

After the emission of the first illumination light for the light emission period of the first illumination light ends, the light source control unit 21 automatically switches illumination light to the second illumination light from the first illumination light. The second illumination light continues to be emitted for only the light emission period of the second illumination light that is set in advance. For example, in a case where the light emission period of the second illumination light is three frames, the second illumination light continues to be emitted for three frames. The second special image obtained from the image pickup of an object to be observed, which is being illuminated with the second illumination light, continues to be displayed on the monitor 18 for only the light emission period of the first illumination light. The automatic switching and emission of the first illumination light and the second illumination light and the switching and display of the first special image and the second special image on the monitor 18 as described above are repeatedly performed until the end of the multi-observation mode, such as the switching of a mode to the other mode.

In the embodiment, the B/G ratio and the G/R ratio are obtained from the first RGB image signals by the signal ratio calculation section 73 and the saturation emphasis processing and the hue emphasis processing are performed in the signal ratio space formed from the B/G ratio and the G/R ratio. However, color information different from the B/G ratio and the G/R ratio may be obtained and the saturation emphasis processing and the hue emphasis processing may be performed in a feature space formed from the color information.

For example, color difference signals Cr and Cb may be obtained as color information and the saturation emphasis processing and the hue emphasis processing may be performed in a feature space formed from the color difference signals Cr and Cb. In this case, a first special image processing unit 100 and a second special image processing unit 101 shown in Fig. 25 are used. Each of the first special image processing unit 100 and the second special image processing unit 101 does not comprise the Log transformation section 72, the signal ratio calculation section 73, and the inverse Log transformation section 83 unlike each of the first special image processing unit 63 and the second special image processing unit 64. Instead, each of the first special image processing unit 100 and the second special image processing unit 101 comprises, a luminance-color difference signal conversion section 104. Other configurations of the first special image processing unit. 100 and the second special image processing unit 101 are the same as those of the first special image processing unit 63 and the second special image processing unit 64.

The luminance-color difference signal conversion section 104 converts the Rlx-image signals, the Glx-image signals, and the Blx-image signals into luminance signals Y and the color difference signals Cr and Cb. A well-known conversion equation is used for the conversion of the signals into the color difference signals Cr and Cb. The color difference signals Cr and Cb are sent to the polar coordinate conversion section 75. The luminance signals Y are sent to the RGB conversion section 79 and the brightness adjustment section 81. The RGB conversion section 79 converts the color difference signals Cr and Cb and the luminance signals Y, which have been transmitted through the orthogonal coordinate conversion section 78, into R1y-image signals, G1y-image signals, and the B1y-image signals.

The brightness adjustment section 81 adjusts the pixel values of the R1y-image signals, the Gly-image signals, and the B1y-image signals by using the luminance signals Y as the first brightness information Yin and using the second brightness information, which is obtained by the second brightness-information calculation section 81b, as the second brightness information Yout. A method of calculating the second brightness information Yout and a method of adjusting the pixel values of the R1y-image signals, the G1y-image signals, and the B1y-image signals are the same as those in the case of the first special image processing unit 63.

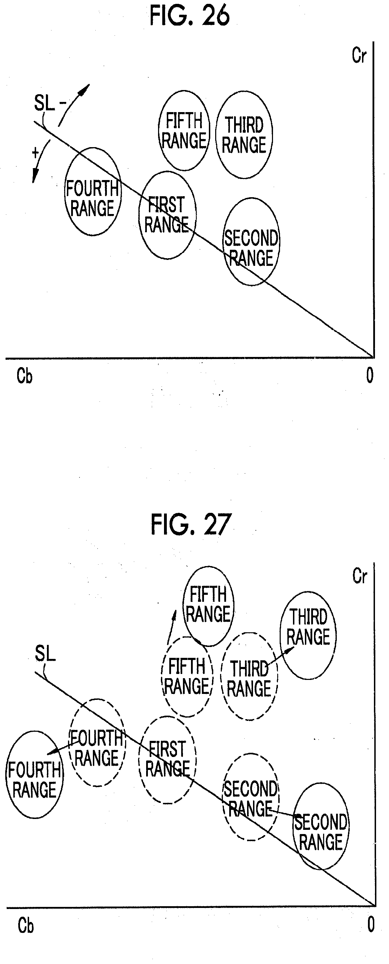

As shown in Fig. 26, the first range including a normal mucous membrane is distributed substantially in the center of the second quadrant of a CrCb space formed from the color difference signals Cr and Cb. The second range including an atrophic mucous membrane is positioned substantially on the clockwise side of a reference line SL passing through the first range including a normal mucous membrane, and is distributed at a position that is closer to the origin than the first range including a normal mucous membrane. The third range including deep blood vessels is distributed on the clockwise side of the reference line SL. The fourth range including a BA is distributed substantially on the counterclockwise side of the reference line SL. The fifth range including redness is distributed on the clockwise side of the reference line SL. The reference line SL corresponds to the above-mentioned hue reference line SLh. In the CrCb space, the counterclockwise direction with respect to the reference line SL corresponds to the above-mentioned positive direction and the clockwise direction with respect to the reference line SL corresponds to the above-mentioned negative direction.

As in the case of the signal ratio space, the saturation emphasis processing for extending or compressing a radius vector r and the hue emphasis processing for increasing or reducing an angle θ are performed in the CrCb space where the first to fifth ranges are distributed as described above. Accordingly, as shown in Fig. 27, a difference between the first range including a normal mucous membrane and the second range (solid line) including an atrophic mucous membrane having been subjected to the saturation emphasis processing and the hue emphasis processing is larger than a difference between the first range including a normal mucous membrane and the second range (dotted line) including an atrophic mucous membrane having not yet been subjected to the saturation emphasis processing and the hue emphasis processing. Likewise, a difference between the first range including a normal mucous membrane and each of deep blood vessels (solid line), the fourth range (solid line) including a BA, and the fifth range (solid line) including redness having been subjected to the saturation emphasis processing and the hue emphasis processing is larger than a difference between the first range including a normal mucous membrane and each of deep blood vessels (dotted line), the fourth range (dotted line) including a BA, and the fifth range (dotted line) including redness having not yet been subjected to the saturation emphasis processing and the hue emphasis processing. Since the color of a mucous membrane of an object to be observed varies depending on a portion, it is preferable that the results of the saturation emphasis processing or the hue emphasis processing based on the color difference signals Cr and Cb are adjusted using adjustment parameters determined for every portion as in the case of the signal ratio space.

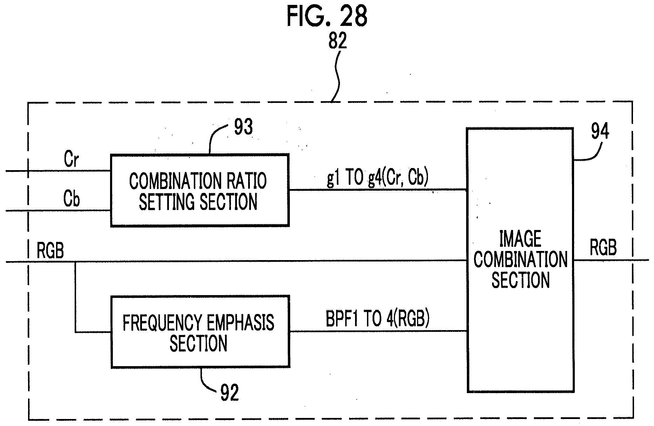

Further, the structure emphasis processing in a case where the color difference signals Cr and Cb are used is also performed by the same method as the method in the case of the signal ratio space. As shown in Fig. 28, the color difference signals Cr and Cb are input to the combination ratio setting section 93 in the structure emphasis section 82 of each of the first special image processing unit 100 and the second special image processing unit 101. The combination ratio setting section 93 sets combination ratios g1(Cr, Cb), g2(Cr, Cb), g3(Cr, Cb), and g4(Cr, Cb), which represent the combination ratios of first to fourth frequency component-emphasized images BPF1(RGB) to BPF4(RGB) with respect to the R1y-image signals, the Gly-image signals, and the B1y-image signals, for every pixel on the basis of Cr and Cb having not yet been subjected to the saturation emphasis processing and the hue emphasis processing. As described above, a method of setting the combination ratios g1(Cr, Cb), g2(Cr, Cb), g3(Cr, Cb), and g4(Cr, Cb) is determined depending on a range, in which Cr and Cb are, among the second to fifth ranges.

Then, the R1y-image signals, the G1y-image signals, and the B1y-image signals are combined with the first to fourth frequency component-emphasized images BPF1(RGB) to BPF4(RGB) according to the combination ratios g1(Cr, Cb), g2(Cr, Cb), g3(Cr, Cb), and g4(Cr, Cb) that are set for every pixel by the combination ratio setting section 93. Accordingly, R1y-image signals, G1y-image signals, and B1y-image signals having been subjected to the structure emphasis processing are obtained. Even in a case where CrCb are used, it is preferable that the pixel values of the R1y-image signals, the G1y-image signals, and the B1y-image signals having been subjected to the structure emphasis processing are adjusted using the adjustment parameters determined for every portion.

Further, a hue H and a saturation S may be obtained as color information and the saturation emphasis processing and the hue emphasis processing may be performed in an HS space formed from the hue H and the saturation S. In a case where a hue H and a saturation S are used, a first special image processing unit 120 and a second special image processing unit 121 shown in Fig. 29 are used. Each of the first special image processing unit 120 and the second special image processing unit 121 does not comprise the Log transformation section 72, the signal ratio calculation section 73, the polar coordinate conversion section 75, the orthogonal coordinate conversion section 78, and the inverse Log transformation section 83 unlike each of the first special image processing unit 63 and the second special image processing unit 64. Instead, each of the first special image processing unit 120 and the second special image processing unit 121 comprises an HSV conversion section 124. Other configurations of the first special image processing unit 120 and the second special image processing unit 121 are the same as those of the first special image processing unit 63 and the second special image processing unit 64.

The HSV conversion section 124 converts the Rlx-image signals, the Glx-image signals, and the Blx-image signals into a hue H, a saturation S, and a brightness value (V). A well-known conversion equation is used for the conversion of the signals into the hue H, the saturation S, and the brightness V. The hue H and the saturation S are sent to the saturation emphasis processing section 76 and the hue emphasis processing section 77. The brightness V is sent to the RGB conversion section 79. The RGB conversion section 79 converts the hue H, the saturation S, and the brightness V, which have been transmitted through the saturation emphasis processing section 76 and the hue emphasis processing section 77, into R1y-image signals, Gly-image signals, and the B1y-image signals.

The brightness adjustment section 81 adjusts the pixel values of the R1y-image signals, the Gly-image signals, and the B1y-image signals by using the first brightness information Yin that is obtained by the first brightness-information calculation section and the second brightness information Yout that is obtained by the second brightness-information calculation section 81b. Methods of calculating the first brightness information Yin and the second brightness information Yout and a method of adjusting the pixel values of the R1y-image signals, the Gly-image signals, and the B1y-image signals are the same as those in the case of the first special image processing unit 63.

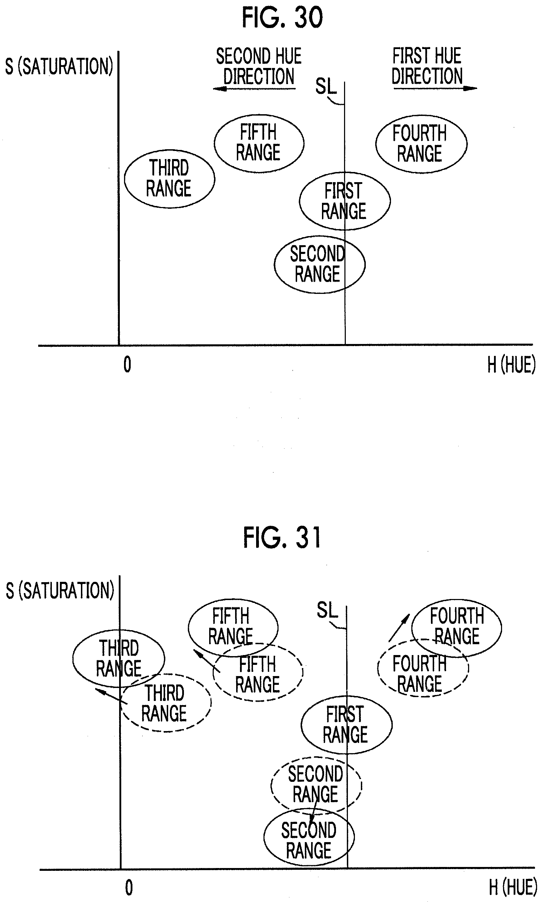

As shown in Fig. 30, the first range including a normal mucous membrane is distributed on a reference line SL, which represents the value of a specific hue, in the HS space formed from the hue H and the saturation S. The second range including an atrophic mucous membrane is distributed at a position where a saturation is lower than the saturation corresponding to the reference line SL. The fourth range including a BA is distributed at a position where a saturation is higher than the saturation of the first range including a normal mucous membrane and which is present on side in a first hue direction (right side) of the reference line SL. The fifth range including redness is distributed at a position where a saturation is higher than the saturation of the first range including a normal mucous membrane and which is present on a side in a second hue direction (left side) of the reference line SL. The third range including deep blood vessels is distributed at a position where a saturation is higher than the saturation of the first range including a normal mucous membrane and is lower than the saturation of the fourth range including a BA or the fifth range including redness. Further, the third range including deep blood vessels is distributed at a position that is present on a side in the second hue direction (left side), which is different from the first hue direction, of the reference line SL. A distance in the hue direction between the fifth range including redness and the reference line SL is shorter than a distance between the third range including deep blood vessels and the reference line SL.

The saturation emphasis processing and the hue emphasis processing to be performed in the HS space where the first to fifth ranges are distributed as described above are processing for translating the second to fifth ranges without increasing and reducing a radius vector r and an angle θ unlike in the signal ratio space and the CrCb space. , As the saturation emphasis processing, the saturation emphasis processing section 76 performs processing for translating the second range including an atrophic mucous membrane in a saturation direction to reduce the saturation of the second range. Further, it is preferable that the saturation emphasis processing section 76 performs processing for translating the third range including deep blood vessels, the fourth range including a BA, and the fifth range including redness in the saturation direction to increase the saturations of the third to fifth ranges, as the saturation emphasis processing.

The third to fifth ranges may be translated so that the saturations of the third to fifth ranges are reduced.

Furthermore, as the hue emphasis processing, the hue emphasis processing section 77 performs processing for translating the third range including deep blood vessels, the fourth range including a BA, and the fifth range including redness in the hue direction so that the third to fifth ranges become far from the first range including a normal mucous membrane. As the hue emphasis processing, the hue emphasis processing section 77 may perform processing for moving the second range including an atrophic mucous membrane in the hue direction.

Since the saturation emphasis processing and the hue emphasis processing having been described above are performed, a difference between the first range including a normal mucous membrane and the second range (solid line) including an atrophic mucous membrane having been subjected to the saturation emphasis processing and the hue emphasis processing is larger than a difference between the first range including a normal mucous membrane and the second range (dotted line) including an atrophic mucous membrane having not yet been subjected to the saturation emphasis processing and the hue emphasis processing as shown in Fig. 31. Likewise, a difference between the first range including a normal mucous membrane and each of deep blood vessels (solid line), the fourth range (solid line) including a BA, and the fifth range (solid line) including redness having been subjected to the saturation emphasis processing and the hue emphasis processing is larger than a difference between the first range including a normal mucous membrane and each of deep blood vessels (dotted line), the fourth range (dotted line) including a BA, and the fifth range (dotted line) including redness having not yet been subjected to the first saturation emphasis processing and the first hue emphasis processing. Since the color of a mucous membrane of an object to be observed varies depending on a portion, it is preferable that the results of the saturation emphasis processing and the hue emphasis processing based on the hue H and the saturation S are adjusted using adjustment parameters determined for every portion as in the case of the signal ratio space.

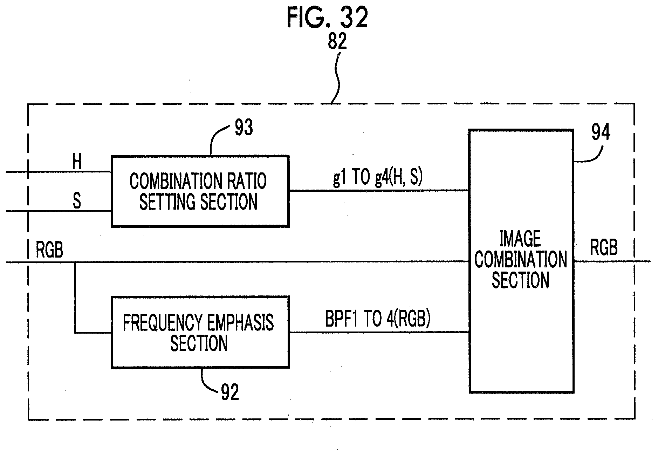

Further, the structure emphasis processing is performed on the basis of CrCb by the same method as that in the case of the signal ratio space. As shown in Fig. 32, the hue H and the saturation S are input to the combination ratio setting section 93 in the structure emphasis section 82 of each of the first special image processing unit 120 and the second special image processing unit 121. The combination ratio setting section 93 sets combination ratios g1(H, S), g2(H, S), g3(H, S), and g4(H, S), which represent the combination ratios of first to fourth frequency component-emphasized images BPF1(RGB) to BPF4(RGB) with respect to the R1y-image signals, the Gly-image signals, and the B1y-image signals, for every pixel on the basis of the hue H and the saturation S having not yet been subjected to the saturation emphasis processing and the hue emphasis processing. As described above, a method of setting the combination ratios g1(H, S), g2(H, S), g3(H, S), and g4(H, S) is determined depending on a range, in which the hue H and the saturation S are, among the second to fifth ranges.

Then, the R1y-image signals, the Gly-image signals, and the B1y-image signals are combined with the first to fourth frequency component-emphasized images BPF1(RGB) to BPF4(RGB) according to the combination ratios g1(H, S), g2(H, S), g3(H, S), and g4(H, S) that are set for every pixel by the combination ratio setting section 93. Accordingly, R1y-image signals, Gly-image signals, and B1y-image signals having been subjected to the structure emphasis processing are obtained. Even in a case where the hue H and the saturation S are used, it is preferable that the pixel values of the R1y-image signals, the Gly-image signals, and the B1y-image signals having been subjected to the structure emphasis processing are adjusted using the adjustment parameters determined for every portion.

[Second embodiment]

In a second embodiment, an object to be observed is illuminated using laser light sources and a fluorescent body instead of the four color LEDs 20a to 20d described in the first embodiment. Others are the same as those of the first embodiment.

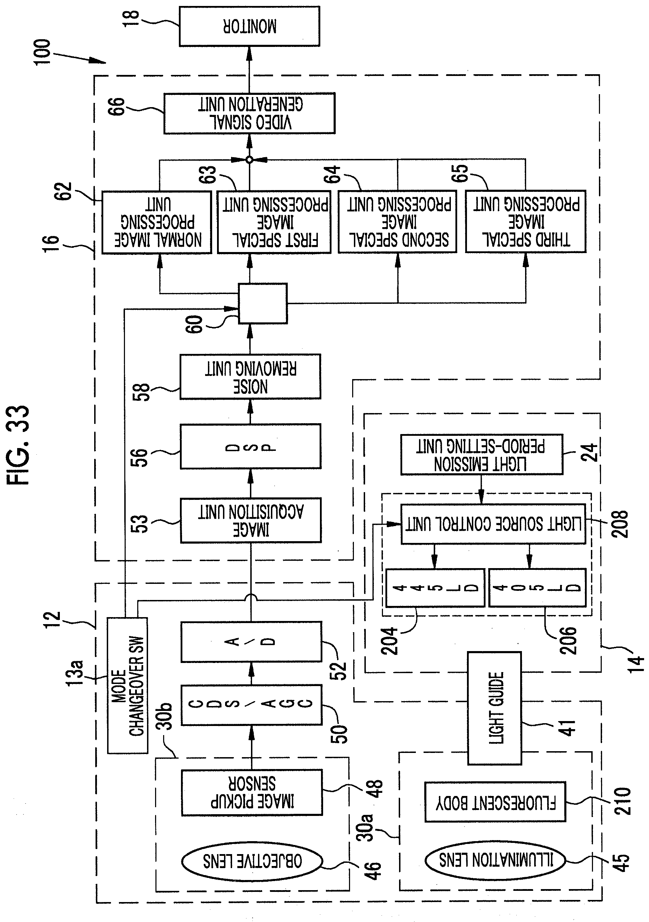

As shown in Fig. 33, in an endoscope system 200 according to a second embodiment, a light source device 14 is provided with a blue laser light source (written in Fig. 33 as "445LD") 204 emitting blue laser light of which the central wavelength is in the range of $445 \pm 10$ nm and a blue-violet laser light source (written in Fig. 33 as "405LD") 206 emitting blue-violet laser light of which the central wavelength is in the range of $405 \pm 10$ nm, instead of the four color LEDs 20a to 20d. Since pieces of light emitted from semiconductor light-emitting elements of the respective light sources 204 and 206. are individually controlled by a light source control unit 208, a ratio of the amount of light emitted from the blue laser light source 204 to the amount of light emitted from the blue-violet laser light source 206 can be freely changed.

The light source control unit 208 drives the blue laser light source 204 in a normal observation mode. In a first special observation mode, the light source control unit 208 drives both the blue laser light source 204 and the blue-violet laser light source 206 and controls blue-violet laser light and blue laser light so that the light emission ratio of blue-violet laser light is higher than the light emission ratio of blue laser light. In a second special observation mode, the light source control unit 208 drives both the blue laser light source 204 and the blue-violet laser light source 206 and controls blue-violet laser light and blue laser light so that the light emission ratio of blue laser light is higher than the light emission ratio of blue-violet laser light.

In a multi-observation mode, the light source control unit 208 drives both the blue laser light source 204 and the blue-violet laser light source 206, controls blue-violet laser light and blue laser light so that the light emission ratio of blue-violet laser light is higher than the light emission ratio of blue laser light in the light emission period of first illumination light, and controls blue-violet laser light and blue laser light so that the light emission ratio of blue laser light is higher than the light emission ratio of blue-violet laser light in the light emission period of second illumination light. Laser light emitted from each of the light sources 204 and 206 is incident on the light guide 41 through optical members (all of the optical members are not shown), such as a condenser lens, optical fibers, or a multiplexer.

It is preferable that the half-width of blue laser light or blue-violet laser light is set to about ±10 nm. Further, broad area-type InGaN-based laser diodes can be used as the blue laser light source 104 and the blue-violet laser light source 106, and InGaNAs-based laser diodes or GaNAs-based laser diodes can also be used. Furthermore, a light emitter, such as a light emitting diode, may be used as the light source.

The illumination optical system 30a is provided with a fluorescent body 210 on which blue laser light or blue-violet laser light transmitted from the light guide 41 is to be incident in addition to the illumination lens 45. In a case where the fluorescent body 210 is irradiated with blue laser light, fluorescence is emitted from the fluorescent body 210. Further, a part of blue laser light passes through the fluorescent body 210 as it is. Blue-violet laser light passes through the fluorescent body 210 without exciting the fluorescent body 210. The inside of a specimen is irradiated with light, which is emitted from the fluorescent body 210, through the illumination lens 45.

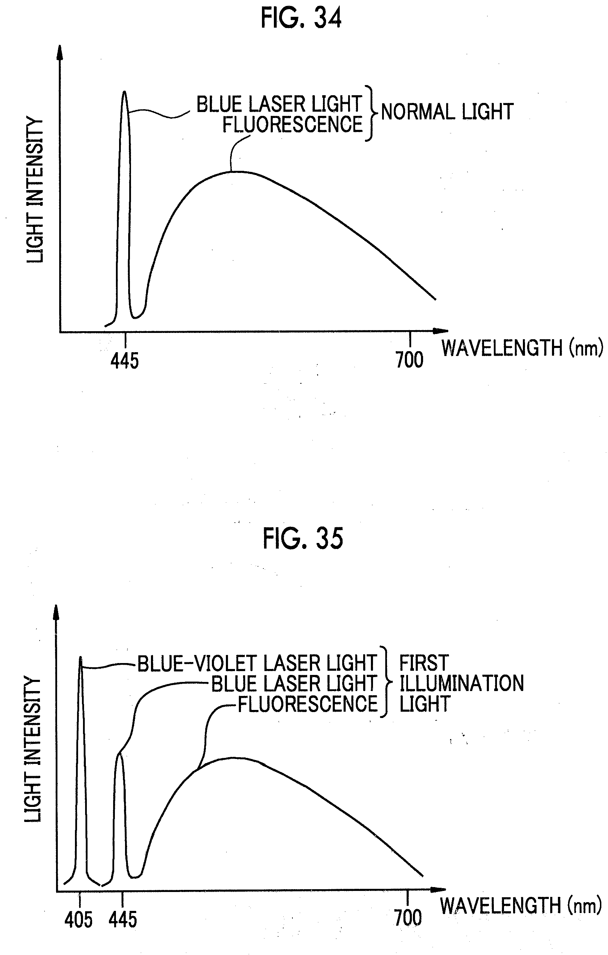

Here, since blue laser light is mainly incident on the fluorescent body 210 in the normal observation mode, an object to be observed is irradiated with normal light shown in Fig. 34 in which blue laser light and fluorescence excited and emitted from the fluorescent body 210 due to blue laser light are multiplexed. Since both blue-violet laser light and blue laser light are incident on the fluorescent body 210 in the first special observation mode, the inside of a specimen is irradiated with first illumination light shown in Fig. 35 in which blue-violet laser light, blue laser light, and fluorescence excited and emitted from the fluorescent body 210 due to blue laser light are multiplexed. In the first illumination light, the light intensity of blue-violet laser light is higher than the light intensity of blue laser light.

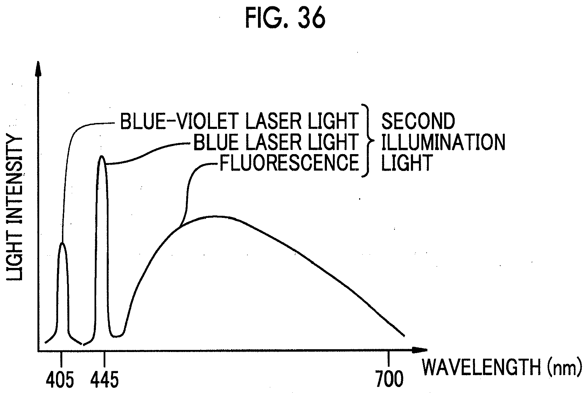

Since both blue-violet laser light and blue laser light are incident on the fluorescent body 210 even in the second special observation mode, the inside of a specimen is irradiated with second illumination light shown in Fig. 36 in which blue-violet laser light, blue laser light, and fluorescence excited and emitted from the fluorescent body 210 due to blue laser light are multiplexed. In the second illumination light, the light intensity of blue laser light is higher than the light intensity of blue-violet laser light. In the multi-observation mode, the first illumination light continues to be emitted for only the light emission period of the first illumination light that is set in advance and the second illumination light then continues to be emitted for only the light emission period of the second illumination light that is set in advance.

It is preferable that a fluorescent body including plural kinds of fluorescent bodies absorbing a part of blue laser light and exciting and emitting green to yellow light (for example, YAG-based fluorescent bodies or fluorescent bodies, such as BAM ($BaMgAl_{10}O_{17}$)) is used as the fluorescent body 210. In a case where the semiconductor light-emitting elements are used as the excitation light source of the fluorescent body 210 as in this example of configuration, high-intensity white light is obtained with high luminous efficiency. Accordingly, not only the intensity of white light can be easily adjusted but also a change in the color temperature and chromaticity of white light can be suppressed to be small.

The hardware structures of the processing units, which are included in the processor device 16 in the embodiments, such as the first special image processing unit 63, the second special image processing unit 64, the first special image processing unit 100, the second special image processing unit 101, the first special image processing unit 120, and the second special image processing unit 121, are various processors to be described below. The various processors include: a central processing unit (CPU) that is a general-purpose processor functioning as various processing units by executing software (program); a programmable logic device (PLD) that is a processor of which circuit configuration can be changed after manufacture, such as a field programmable gate array (FPGA); a dedicated electrical circuit that is a processor having circuit configuration designed exclusively to perform various kinds of processing; and the like.

One processing unit may be formed of one of these various processors, or may be formed of a combination of two or more same kind or different kinds of processors (for example, a plurality of FPGAs or a combination of a CPU and an FPGA). Further, a plurality of processing units may be formed of one processor. As an example where a plurality of processing units are formed of one processor, first, there is an aspect where one processor is formed of a combination of one or more CPUs and software as typified by a computer, such as a client or a server, and functions as a plurality of processing units. Second, there is an aspect where a processor fulfilling the functions of the entire system, which includes a plurality of processing units, by one integrated circuit (IC) chip as typified by System On Chip (SoC) or the like is used. In this way, various processing units are formed using one or more of the above-mentioned various processors as hardware structures.

In addition, the hardware structures of these various processors are more specifically electrical circuitry where circuit elements, such as semiconductor elements, are combined.

The invention can be applied to various medical image processing devices other than the processor device that is to be combined with the endoscope systems described in the first to third embodiments or a capsule endoscope system disclosed in the fourth embodiment.

Explanation of References

10: endoscope system
12: endoscope
12a: insertion part
12b: operation part
12c: bendable part
12d: distal end part
12e: angle knob
14: light source device
16: processor device
18: monitor
19: console
20: light source unit
20a: V-LED
20b: B-LED
20c: G-LED
20d: R-LED
21: light source control unit
23: optical path-combination unit
24: light emission period-setting unit
26a: slide bar
26b: slide bar
27a: slider
27b: slider
30a: illumination optical system
30b: image pickup optical system
41: light guide
45: illumination lens
46: objective lens
48: image pickup sensor
50: CDS/AGC circuit
52: A/D converter
53: image acquisition unit
56: DSP
58: noise removing unit
60: signal switching unit
62: normal image processing unit
63: first special image processing unit
64: second special image processing unit
66: video signal generation unit
70: reverse gamma conversion section
71: specific color adjustment section
72: Log transformation section
73: signal ratio calculation section
75: polar coordinate conversion section
76: saturation emphasis processing section
77: hue emphasis processing section
78: orthogonal coordinate conversion section
79: RGB conversion section
81: brightness adjustment section
81a: first brightness-information calculation section
81b: second brightness-information calculation section
82: structure emphasis section
83: inverse Log transformation section
84: gamma conversion section

86: portion setting section

90: specific color adjustment section

92: frequency emphasis section

93: combination ratio setting section

94: image combination section

100: first special image processing unit

101: second special image processing unit

104: luminance-color difference signal conversion section

104: blue laser light source

106: blue-violet laser light source

120: first special image processing unit

121: second special image processing unit

124: HSV conversion section

200: endoscope system

204: blue laser light source

204: blue laser light source

206: blue-violet laser light source

208: light source control unit

210: fluorescent body

**Claims**

1. A light source device comprising:

   a light source unit that emits first illumination light including a first red-light wavelength range and used to emphasize a first blood vessel and second illumination light including a second red-light wavelength range and used to emphasize a second blood vessel different from the first blood vessel; and
   a light source control unit that causes each of the first illumination light and the second illumination light to be emitted for a light emission period of at least two or more frames and automatically switches the first illumination light and the second illumination light.

2. The light source device according to claim 1,
   wherein the first illumination light has a peak in a range of 400 nm to 440 nm.

3. The light source device according to claim 1 or 2,
   wherein an intensity ratio of the second illumination light is higher than that of the first illumination light in at least one of wavelengths of 540 nm, 600 nm, or 630 nm.

4. The light source device according to any one of claims 1 to 3,

   wherein the light source unit emits third illumination light different from the first illumination light and the second illumination light, and
   the light source control unit causes the third illumination light to be emitted at a timing of the switching of the first illumination light and the second illumination light.

5. The light source device according to any one of claims 1 to 4,
   wherein the light source control unit includes a light emission period-setting unit that sets a light emission period of the first illumination light and a light emission period of the second illumination light.

6. The light source device according to any one of claims 1 to 5,

   wherein the first illumination light includes a first green-light wavelength range and a first blue-light wavelength range in addition to the first red-light wavelength range, and
   the second illumination light includes a second green-light wavelength range and a second blue-light wavelength range in addition to the second red-light wavelength range.

7. An endoscope system comprising:

the light source device according to any one of claims 1 to 6;
an image acquisition unit that acquires a first image obtained from image pickup of an object to be observed illuminated with the first illumination light and a second image obtained from image pickup of the object to be observed illuminated with the second illumination light; and
a display unit that displays the first image and the second image as color images or monochrome images.

8. An endoscope system comprising:

the light source device according to any one of claims 1 to 6;
an image acquisition unit that acquires a first image obtained from image pickup of an object to be observed illuminated with the first illumination light and a second image obtained from image pickup of the object to be observed illuminated with the second illumination light; and
a specific color adjustment section that adjusts colors of the first image and the second image,
wherein the specific color adjustment section causes a color of a mucous membrane included in the first image or a color of a mucous membrane included in the second image to match a target color.

9. The endoscope system according to claim 8, further comprising:

a portion setting section that sets a portion being observed,
wherein the specific color adjustment section causes the color of the mucous membrane included in the first image or the color of the mucous membrane included in the second image to match a target color corresponding to the portion.

10. An endoscope system comprising:

the light source device according to any one of claims 1 to 6;
an image acquisition unit that acquires a first image obtained from image pickup of an object to be observed illuminated with the first illumination light and a second image obtained from image pickup of the object to be observed illuminated with the second illumination light;
a specific color adjustment section that adjusts colors of the first image and the second image; and
a color adjustment instruction-receiving unit that receives a color adjustment instruction related to adjustment of a color of a mucous membrane, the color of the first blood vessel, or the color of the second blood vessel,
wherein the specific color adjustment section adjusts the color of the mucous membrane, a color of the first blood vessel, or a color of the second blood vessel according to the color adjustment instruction from a user.

11. An endoscope system comprising:

the light source device according to any one of claims 1 to 6;
an image acquisition unit that acquires a first image obtained from image pickup of an object to be observed illuminated with the first illumination light and a second image obtained from image pickup of the object to be observed illuminated with the second illumination light; and
a specific color adjustment section that adjusts colors of the first image and the second image,
wherein the specific color adjustment section causes a color of a mucous membrane included in the first image to coincide with a color of a mucous membrane included in the second image.

12. An endoscope system comprising:

the light source device according to any one of claims 1 to 6;
an image acquisition unit that acquires a first image obtained from image pickup of an object to be observed illuminated with the first illumination light and a second image obtained from image pickup of the object to be observed illuminated with the second illumination light; and
a color extension processing section that performs color extension processing for increasing a difference between a plurality of ranges included on the object to be observed in the first image and the second image.

13. The endoscope system according to claim 12, further comprising:

a portion setting section that sets a portion being observed, and
wherein a result of the color extension processing is adjusted using an adjustment parameter determined for each portion.

14. An endoscope system comprising:

the light source device according to any one of claims 1 to 6;
an image acquisition unit that acquires a first image obtained from image pickup of an object to be observed illuminated with the first illumination light and a second image obtained from image pickup of the object to be observed illuminated with the second illumination light;
a frequency emphasis section that obtains a frequency-emphasized image, in which a frequency component corresponding to a specific range included in the object to be observed is emphasized, from the first image and the second image; and
an image combination section that combines the first image or the second image with the frequency-emphasized image to obtain the first image or the second image which has been subjected to structure emphasis processing in which the specific range is subjected to structure emphasis.

15. The endoscope system according to claim 14, further comprising:

a portion setting section that sets a portion being observed,
wherein a pixel value of the first image or the second image having been subjected to the structure emphasis processing is adjusted using an adjustment parameter determined for each portion.

# FIG. 1

FIG. 2

10

18 — MONITOR

16

66 — VIDEO SIGNAL GENERATION UNIT

62 — NORMAL IMAGE PROCESSING UNIT

63 — FIRST SPECIAL IMAGE PROCESSING UNIT

64 — SECOND SPECIAL IMAGE PROCESSING UNIT

65 — THIRD SPECIAL IMAGE PROCESSING UNIT

60

58 — NOISE REMOVING UNIT

56 — D S P

53 — IMAGE ACQUISITION UNIT

24 — LIGHT EMISSION PERIOD-SETTING UNIT

21 — LIGHT SOURCE CONTROL UNIT

20 — V-LED (20a), B-LED (20b), G-LED (20c), R-LED (20d)

23 — OPTICAL PATH-COMBINATION UNIT

14

12

13a — MODE CHANGEOVER SW

52 — A / D

50 — CDS / AGC

30b

48 — IMAGE PICKUP SENSOR

30a

46 — OBJECTIVE LENS

41 — LIGHT GUIDE

45 — ILLUMINATION LENS

FIG. 3

FIG. 4

# FIG. 5

# FIG. 6

# FIG. 7

LIGHT EMISSION PERIOD-SETTING MENU

FIRST ILLUMINATION LIGHT:    2
(FRAME)

2 ●————————————● 10   ← 26a

△—27a

SECOND ILLUMINATION LIGHT:    3
(FRAME)

2 ●————————————● 10   ← 26b

△—27b

# FIG. 8

# FIG. 9

18

FIRST SPECIAL IMAGE

# FIG. 10

18

SECOND SPECIAL IMAGE

# FIG. 11

ONE FRAME

FIRST ILLUMINATION LIGHT — FIRST ILLUMINATION LIGHT — SECOND ILLUMINATION LIGHT — SECOND ILLUMINATION LIGHT — SECOND ILLUMINATION LIGHT — FIRST ILLUMINATION LIGHT — FIRST ILLUMINATION LIGHT

FIRST SPECIAL IMAGE (COLOR) — FIRST SPECIAL IMAGE (COLOR) — SECOND SPECIAL IMAGE (COLOR) — SECOND SPECIAL IMAGE (COLOR) — SECOND SPECIAL IMAGE (COLOR) — FIRST SPECIAL IMAGE (COLOR) — FIRST SPECIAL IMAGE (COLOR)

TIME

EP 3 673 787 A1

FIG. 12

FIG. 13

# FIG. 14

FIRST SPECIAL IMAGE PROCESSING UNIT 63

86
PORTION SETTING SECTION

70 REVERSE GAMMA CONVERSION SECTION

71 SPECIFIC COLOR ADJUSTMENT SECTION

72 Log TRANSFORMATION SECTION

73 SIGNAL RATIO CALCULATION SECTION

B/G RATIO
G/R RATIO

75 POLAR COORDINATE CONVERSION SECTION

r
θ

76 SATURATION EMPHASIS PROCESSING SECTION

77 HUE EMPHASIS PROCESSING SECTION

COLOR EXTENSION PROCESSING SECTION

78 ORTHOGONAL COORDINATE CONVERSION SECTION

B/G RATIO
G/R RATIO

79 RGB CONVERSION SECTION

81 BRIGHTNESS ADJUSTMENT SECTION
81a 81b

82 STRUCTURE EMPHASIS SECTION

83 INVERSE Log TRANSFORMATION SECTION

84 GAMMA CONVERSION SECTION

SECOND SPECIAL IMAGE PROCESSING UNIT 64

90 SPECIFIC COLOR ADJUSTMENT SECTION

B
G
R

EP 3 673 787 A1

# FIG. 15

B/G RATIO

FOURTH RANGE

FIRST RANGE

FIFTH RANGE

THIRD RANGE

SECOND RANGE

+ SL

−

0                                              G/R RATIO

# FIG. 16

B/G RATIO

r2

rc

r1

SLs

RADIUS VECTOR r

rcr1

rcr2

Rm

0                                              G/R RATIO

## FIG. 17

## FIG. 18

## FIG. 19

## FIG. 20

# FIG. 21

# FIG. 22

82

# FIG. 23

| | | g1(B/G RATIO, G/R RATIO) | g2(B/G RATIO, G/R RATIO) | g3(B/G RATIO, G/R RATIO) | g4(B/G RATIO, G/R RATIO) |
|---|---|---|---|---|---|
| B/G RATIO, G/R RATIO | SECOND RANGE | g1x | g2y | g3y | g4y |
| | THIRD RANGE | g1y | g2x | g3y | g4y |
| | FOURTH RANGE | g1y | g2y | g3x | g4y |
| | FIFTH RANGE | g1y | g2y | g3y | g4x |
| | OTHER THAN SECOND TO FIFTH RANGES | g1y | g2y | g3y | g4y |

## FIG. 24

```
                    ( START )
                        |
     ┌──────────────────────────────────────┐
     │        MULTI-OBSERVATION MODE         │
     └──────────────────────────────────────┘
                        |
     ┌──────────────────────────────────────┐
     │    EMIT FIRST ILLUMINATION LIGHT FOR  │
     │    LIGHT EMISSION PERIOD OF FIRST     │
     │         ILLUMINATION LIGHT            │
     └──────────────────────────────────────┘
                        |
     ┌──────────────────────────────────────┐
     │     DISPLAY FIRST SPECIAL IMAGE FOR   │
     │    LIGHT EMISSION PERIOD OF FIRST     │
     │         ILLUMINATION LIGHT            │
     └──────────────────────────────────────┘
                        |
     ┌──────────────────────────────────────┐
     │  EMIT SECOND ILLUMINATION LIGHT FOR   │
     │   LIGHT EMISSION PERIOD OF SECOND     │
     │         ILLUMINATION LIGHT            │
     └──────────────────────────────────────┘
                        |
     ┌──────────────────────────────────────┐
     │  DISPLAY SECOND SPECIAL IMAGE FOR     │
     │   LIGHT EMISSION PERIOD OF SECOND     │
     │         ILLUMINATION LIGHT            │
     └──────────────────────────────────────┘
                        |
    <  DOES MULTI-OBSERVATION MODE END?  >
  N                     |
                        | Y
                    ( END )
```

# FIG. 25

EP 3 673 787 A1

## FIG. 26

## FIG. 27

# FIG. 28

82

| Cr | COMBINATION RATIO SETTING SECTION (93) | g1 TO g4(Cr, Cb) | IMAGE COMBINATION SECTION (94) | RGB |

RGB

FREQUENCY EMPHASIS SECTION (92) — BPF1 TO 4(RGB)

# FIG. 29

FIRST SPECIAL IMAGE PROCESSING UNIT — 120

- 86 PORTION SETTING SECTION
- 70 REVERSE GAMMA CONVERSION SECTION
- 71 SPECIFIC COLOR ADJUSTMENT SECTION
- 124 HSV CONVERSION SECTION
- 76 SATURATION EMPHASIS PROCESSING SECTION
- 77 HUE EMPHASIS PROCESSING SECTION
- COLOR EXTENSION PROCESSING SECTION
- 79 RGB CONVERSION SECTION
- 81 BRIGHTNESS ADJUSTMENT SECTION (81a, 81b)
- 82 STRUCTURE EMPHASIS SECTION
- 84 GAMMA CONVERSION SECTION

SECOND SPECIAL IMAGE PROCESSING UNIT — 121

- 90 SPECIFIC COLOR ADJUSTMENT SECTION

EP 3 673 787 A1

## FIG. 30

## FIG. 31

## FIG. 32

82

# FIG. 33

EP 3 673 787 A1

## FIG. 34

## FIG. 35

## FIG. 36

**INTERNATIONAL SEARCH REPORT**

| | International application No. |
|---|---|
| | PCT/JP2018/030291 |

A. CLASSIFICATION OF SUBJECT MATTER
Int.Cl.     A61B1/06(2006.01)i,   A61B1/045(2006.01)i,   G02B23/24(2006.01)i,
              G02B23/26(2006.01)i
According to International Patent Classification (IPC) or to both national classification and IPC

B. FIELDS SEARCHED

Minimum documentation searched (classification system followed by classification symbols)
Int.Cl. A61B1/06, A61B1/045, G02B23/24, G02B23/26

Documentation searched other than minimum documentation to the extent that such documents are included in the fields searched
      Published examined utility model applications of Japan          1922–1996
      Published unexamined utility model applications of Japan        1971–2018
      Registered utility model specifications of Japan                1996–2018
      Published registered utility model applications of Japan        1994–2018

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | WO 2017/061003 A1 (OLYMPUS CORP.) 13 April 2017, paragraphs [0029]-[0481], fig. 1-29 & CN 108135459 A | 1-15 |
| Y | JP 2010-68992 A (FUJIFILM CORP.) 02 April 2010, paragraphs [0005]-[0017], [0054]-[0061], fig. 5 & US 2010/0069713 A1, paragraphs [0007]-[0016], [0054]-[0057], fig. 5 | 1-15 |
| Y | JP 2016-131837 A (FUJIFILM CORP.) 25 July 2016, paragraph [0036] (Family: none) | 5-15 |

☒ Further documents are listed in the continuation of Box C.      ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 19 October 2018 (19.10.2018) | 30 October 2018 (30.10.2018) |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

International application No.

PCT/JP2018/030291

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2005-296200 A (OLYMPUS CORP.) 27 October 2005, paragraphs [0015]-[0135], fig. 1-20 (Family: none) | 8-10 |
| Y | JP 2006-341076 A (OLYMPUS MEDICAL SYSTEMS CORP.) 21 December 2006, paragraphs [0008]-[0134], fig. 11-22 & US 8279275 B2, column 4, line 66 to column 22, line 18, fig. 11-22 & WO 2006/120794 A1 & EP 1880658 A1 & CA 2608294 A1 & KR 10-2008-0007582 A & CN 101163438 A | 8-10 |
| Y | WO 2010/044432 A1 (OLYMPUS MEDICAL SYSTEMS CORP.) 22 April 2010, paragraphs [0018]-[0051], fig. 14A-14B & US 2010/0331624 A1, paragraphs [0061]-[0097], fig. 14A-14B & EP 2335560 A1 & CN 102170817 A | 9, 13, 15 |
| Y | JP 2009-77765 A (FUJIFILM CORP.) 16 April 2009, paragraphs [0054]-[0060] (Family: none) | 11 |
| Y | WO 2015/064435 A1 (FUJIFILM CORP.) 07 May 2015, paragraphs [0019]-[0092], fig. 1-14 & US 2016/0239965 A1, paragraphs [0043]-[0114], fig. 1-14 & EP 3072439 A1 & CN 108109134 A | 12-15 |

Form PCT/ISA/210 (continuation of second sheet) (January 2015)

52

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016179236 A